(19) **Europäisches Patentamt / European Patent Office / Office européen des brevets**

(11) **EP 4 329 590 B1**

(12) **EUROPEAN PATENT SPECIFICATION**

(45) Date of publication and mention
of the grant of the patent:
**05.11.2025   Bulletin 2025/45**

(21) Application number: **22722283.3**

(22) Date of filing: **18.04.2022**

(51) International Patent Classification (IPC):
*A61B 5/00* (2006.01)    *A61B 5/055* (2006.01)
*A61B 5/022* (2006.01)    *A61B 8/06* (2006.01)
*G16H 50/50* (2018.01)    *A61B 5/021* (2006.01)
*A61B 5/02* (2006.01)    *A61B 8/02* (2006.01)
*A61B 8/04* (2006.01)    *A61B 8/08* (2006.01)
*A61B 8/00* (2006.01)    *A61B 6/50* (2024.01)

(52) Cooperative Patent Classification (CPC):
**A61B 5/7267; A61B 5/02028; A61B 5/02208;
A61B 5/055; A61B 8/06; A61B 8/065;
A61B 8/0883; A61B 8/5223; G16H 50/50;**
A61B 6/503; A61B 6/504; A61B 6/507; A61B 8/02;
A61B 8/04; A61B 8/5207

(86) International application number:
**PCT/EP2022/060202**

(87) International publication number:
**WO 2022/228928 (03.11.2022 Gazette 2022/44)**

(54) **SYSTEM AND METHOD FOR ESTIMATING VALUE FOR TARGET CARDIAC PARAMETER**

SYSTEM UND VERFAHREN ZUR SCHÄTZUNG DES WERTS FÜR EINEN ZIELHERZPARAMETER

SYSTÈME ET PROCÉDÉ D'ESTIMATION DE VALEUR POUR PARAMÈTRE CARDIAQUE CIBLE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **27.04.2021   US 202163180217 P
07.02.2022   EP 22155385**

(43) Date of publication of application:
**06.03.2024   Bulletin 2024/10**

(73) Proprietor: **Koninklijke Philips N.V.
5656 AG Eindhoven (NL)**

(72) Inventors:
• **TOKOUTSI, Zoi
5656 AG Eindhoven (NL)**
• **NOERTEMANN, Folke Charlotte
5656 AG Eindhoven (NL)**
• **LAVEZZO, Valentina
5656 AG Eindhoven (NL)**
• **LAU, Kevin Daniel Seng Hung
5656 AG Eindhoven (NL)**

• **MAESSEN, Ralph Theodorus Hubertus
5656 AG Eindhoven (NL)**

(74) Representative: **Philips Intellectual Property &
Standards
High Tech Campus 52
5656 AG Eindhoven (NL)**

(56) References cited:
**EP-A2- 3 043 276    WO-A2-2014/162181**

• **GILBERT M G ET AL: "INTEGRATED
STRUCTURE/CONTROL LAW DESIGN BY
MULTILEVEL OPTIMIZATION", JOURNAL OF
GUIDANCE AND CONTROL AND DYNAMICS,
AIAA, RESTON, VA, US, vol. 14, no. 5, 1
September 1991 (1991-09-01), pages 1001 - 1007,
XP000320662, ISSN: 0731-5090**

- **ITU LUCIAN ET AL: "Model based non-invasive estimation of PV loop from echocardiography", 2014 36TH ANNUAL INTERNATIONAL CONFERENCE OF THE IEEE ENGINEERING IN MEDICINE AND BIOLOGY SOCIETY, IEEE, 26 August 2014 (2014-08-26), pages 6774 - 6777, XP032675249, DOI: 10.1109/EMBC.2014.6945183**

**Description**

FIELD OF THE INVENTION

The invention relates to the field of cardiac parameters, and in particular to estimating a value of a target cardiac parameter.

BACKGROUND OF THE INVENTION

Cardiac parameters, such as cardiac filling pressures, are important measurements for both diagnostic (e.g. for heart failure with preserved ejection fraction, HFpEF) and monitoring purposes, providing information about left and right heart function, and cardiovascular and cardiopulmonary interactions. High cardiac filling pressures are associated with myocardial dysfunction and/or hypervolemia, and subjects with high cardiac filling pressures tend to have poor long-term survival rates.

Cardiac filling pressure measurements are typically obtained by left heart catheterization for left ventricular pressure or left ventricular filling pressure, LVFP, and right heart catheterization for pulmonary capillary wedge pressure, PCWP. The invasive nature of catheterization means that conventional approaches for obtaining these measurements are uncomfortable, costly, time-consuming, and present an additional risk to the patient. It can also be logistically difficult to schedule taking these measurements in a heart failure diagnostic process. Due to these drawbacks, cardiac filling pressures have historically only been used in the diagnosis of heart failure in cases in which there is ambiguity in the diagnosis.

Various studies have explored the relationship of non-invasive measurements with such cardiac filling pressure measurements. However, reproducibility of identified relationships in literature has proven elusive, such that the reliability, robustness or effectiveness of any statistical models that exploit any such relationships has been brought into question. It is believed that any such existing statistical models are heavily dependent on the patient population and the user/measurement accuracy.

There is therefore a need for an improved method for estimating a value for a target cardiac parameter, such as a left ventricular filling pressure.

EP 3,043,276 A2 discloses a personalized whole-body circulation calculation. A combination of models at different scales and machine learning can be used to personalize and calculate the circulation for a particular patient. Imaging, ECG, and pressure data can be used to personalize the multi-scale whole-body circulation model. Different parameters, such as time-varying flow rate for the heart, pressure variation for the heart, cardiovascular systemic impedance, and cardiovascular pulmonary impedance, are determined for the patient and used to personalize the model. The model is then used to determine, visualize or report a diagnostically or therapeutically useful circulation metric for that patient.

SUMMARY OF THE INVENTION

The invention is defined by the claims.

According to examples in accordance with an aspect of the invention, there is provided a processing system for estimating a set of one or more values for a target cardiac parameter of a subject.

The processing system is configured to: obtain a model of a cardiovascular system that uses a plurality of model parameters to produce output data comprising a plurality of output value sets, each associated with a different non-invasively measurable property of cardiac function, wherein: each output value set contains at least one value of the associated non-invasively measurable property of cardiac function; and the plurality of model parameters includes the target cardiac parameter and/or the output data includes a further output value set for the target cardiac parameter; obtain cardiac data comprising a plurality of measured value sets, wherein each measured value set is associated with a different output value set and comprises one or more measured values for the non-invasively measurable property associated with the associated output value set; iteratively modify the values of the plurality of model parameters of the model to thereby modify a difference between the plurality of output value sets of the output data and the corresponding measured value sets in the cardiac data until one or more predetermined criteria are met, wherein the processing system is configured to iteratively modify the values of the plurality of model parameters of the model by: dividing the plurality of model parameters into a plurality of sets of one or more model parameters; setting an initial value set for each of the plurality of model parameters; for each set of one or more model parameters in turn, performing iterative steps of: determining one or more output value sets of the model of the cardiovascular system based on a current value set for at least the set of model parameters; calculating a value of a cost function based on the determined one or more output value sets and the measured value sets associated with the one or more output value sets; and adjusting the value set for each model parameter in the set of model parameters based on the calculated value of the cost function, wherein the iterative steps are repeated until a predefined convergence condition is fulfilled; and following the iterative modification, either: if the plurality of model parameters includes the target cardiac parameter, define the set of one or more values of the modified target cardiac parameter as the estimated set of one or more values for the target cardiac parameter; or if the output data includes the further output value set, define the further output value set as the estimated set of one or more values for the target cardiac parameter.

The inventors have recognized that a model of the cardiovascular system (CVS) may be used to estimate the value or values of a target cardiac parameter non-invasively. The model is personalized to the cardiac function of the subject by fitting model parameters based on non-invasively acquired measurements of the subject. This avoids the need for heart catheterization, thus reducing a risk, cost and time of obtaining a left ventricular filling pressure.

The CVS model is a biophysical model of (parts of) the function of the heart and circulatory system. For any defined set of values for each of a plurality of model parameters, the CVS model outputs a solution (i.e. output data) that includes output value sets of the plurality of non-invasive measurement parameters. The values of the plurality of model parameters are modified until the output value sets match (e.g. within a predetermined error or certainty) the measurement value sets contained in the subject's cardiac data.

Non-invasive measurement indicators representative of cardiac function are parameters representative of cardiac function that can be measured using non-invasive methods (e.g. echocardiography, cardiac MRI, cuff pressure measurements, etc.). Approaches for non-invasively acquiring such measurements are well known and established in the art.

Following the iterative modification, the modified value set of each model parameter is thereby personalized or tailored towards the subject under investigation, i.e. is a subject-specific value. This means that a value set for a target cardiac parameter can be obtained. This value set may be contained in the output data provided by the CVS model and/or in the model parameter of the CVS model.

The target cardiac parameter may be a left ventricular pressure. This embodiment provides a particularly useful scenario for using the proposed approach, as existing methods of determining a left ventricular pressure rely upon invasive measurements, and it would be preferable to generate an accurate measure of this value (for improved assessment of the subject) without the need for surgical intervention.

The plurality of non-invasively measurable properties may comprise a left ventricular volume and at least one peripheral pressure property. These parameters alone may be sufficient to determine the values of the model parameters that most closely correspond to the subject's cardiac data.

A left ventricular volume may, for example, be measured based on 2D echocardiography (using the method of discs), 3D echocardiography or cardiac MRI.

A peripheral pressure may, for example, be measured by taking cuff pressure measurement during diastole and systole (e.g. to measure a brachial, finger, neck toe, ankle and/or leg pressure), or using ultrasound (e.g. to measure an aortic pressure).

The at least one peripheral pressure measurement may comprise a pressure measurement from an arm, leg, wrist, neck, foot, finger or toe of the subject. In one example, the peripheral pressure measurement may, for instance, be a brachial cuff pressure (e.g. measured at an arm of the subject). As another example, the peripheral pressure measurement may be a measurement taken at a neck of the subject, e.g. a carotid pressure.

In some examples, the peripheral pressure measurement may be converted to an aortic pressure, e.g. using one or more transfer functions.

The plurality of non-invasively measurable indicators may comprise at least one of: an aortic valve flow, a mitral valve flow and/or a timing of a cardiac cycle event.

These measurements may be used to improve an accuracy of the estimate, e.g. compared to a left ventricular volume and at least one peripheral pressure measurement alone.

Aortic and/or mitral valve flow measurements may, for example, be determined using cardiovascular MRI or based on Doppler measurements and valve orifice area.

A timing of a cardiac cycle event may, for example, comprise a timing of an opening of a mitral valve, a timing of a closing of a mitral valve, a timing of an opening of an aortic valve, a timing of a closing of an aortic valve, and/or a timing of an onset of left ventricular contraction. Such measurements may be determined from ECG data.

The processing system may be configured to iteratively modify the plurality of model parameters by: defining a solution vector comprising the plurality of output value sets of the model of the cardiovascular system; defining a measurement data vector comprising the plurality of measured value sets; defining a cost function that quantifies a difference between the solution vector and the measurement data vector; and iteratively determining a value of the cost function and modifying the plurality of model parameters based on the value of the cost function until the one or more predetermined criteria are met.

The values for the model parameters for which the cost function is minimized are the values at which the output of the CVS model most closely matches the subject's cardiac data.

The processing system may be configured to iteratively determine a value of the cost function and modify the plurality of model parameters by: setting an initial value set for each of the plurality of model parameters; determining the plurality of output value sets of the model of the cardiovascular system based on a current value set for each of the plurality of model parameters; calculating a value of the cost function based on the determined plurality of output values; modifying the value set for each of the plurality of model parameters based on the calculated value of the cost function; and iteratively repeating the steps of determining the plurality of output value sets, calculating the value of the cost function, and modifying the value set for each of the plurality of model parameters until the one or more predetermined criteria are met. In other words, an all-at-once approach may be used to minimize the cost function. This allows the subject-specific values to be identified efficiently.

values of the model parameters may, for example, be adjusted using a gradient-based minimization method (e.g. the BFGS method) or a non-gradient-based method (e.g. particle swarm or Bayesian optimization).

In some examples, the processing system is configured to iteratively modify the values of the plurality of model parameters of the model by: dividing the plurality of model parameters into a plurality of sets of one or more model parameters; setting an initial value set for each of the plurality of model parameters; for each set of one or more model parameters in turn, performing iterative steps of: determining one or more output value sets of the model of the cardiovascular system based on a current value set for at least the set of model parameters; calculating a value of a cost function based on the determined one or more output value sets and the measured value sets associated with the one or more output value sets; and adjusting the value set for each model parameter in the set of model parameters based on the calculated value of the cost function, wherein the iterative steps are repeated until a predefined convergence condition is fulfilled,

The overall process of iteratively modifying each set of one or more model parameters in turn may be iteratively repeated until one or more predetermined criteria are met.

The processing system may be configured to: divide the plurality of model parameters into a plurality of sets of one or more model parameters by dividing the plurality of model parameters into a first set of model parameters and a second set of model parameters; modify a value set for each of the first set of model parameters by: determining the plurality of output value sets of the model of the cardiovascular system based on a current value set for each of the plurality of model parameters; calculating a value of the cost function based on the determined plurality of output value sets; adjusting the value set for each of the first set of model parameters based on the calculated value of the cost function; and iteratively repeating the steps of determining the plurality of output value sets, calculating the value of the cost function, and adjusting the value set for each of the first set of model parameters until a first predefined convergence condition is fulfilled; modify a value set for each of the second set of model parameters by: determining the plurality of output value sets of the model of the cardiovascular system based on a current value set for each of the plurality of model parameters; calculating a value of the cost function based on the determined plurality of output value sets; adjusting the value set for each of the second set of model parameters based on the calculated value of the cost function; and iteratively repeating the steps of determining the plurality of output value sets, calculating the value of the cost function, and adjusting the value set for each of the second set of model parameters until a second predefined convergence condition is fulfilled; and iteratively repeat the steps of modifying a value set for each of the first set of model parameters and modifying a value set for each of the second set of model parameters until the one or more predetermined criteria are met.

In other words, a bilevel optimization approach, which optimizes different groups of parameters independently of each other, may be used to minimize the cost function. The use of a bilevel optimization algorithm reduces a likelihood of the optimization process getting stuck at a local minimum on the cost function hypersurface and therefore not identifying the global minimum.

The plurality of model parameters may include the target cardiac parameter and the processing system is configured to divide the plurality of model parameters into a first set of model parameters and a second set of model parameters by: defining the first set of model parameters to comprise only the target cardiac parameter; and defining the second set of model parameters to comprise the remaining model parameters. In this way, the target cardiac parameter is optimized independently of the other model parameters.

The processing system may be configured to divide the plurality of model parameters into a first set of model parameters and a second set of model parameters by: performing a sensitivity analysis on the model of the cardiovascular system; and dividing the plurality of model parameters into the first set and second set based on the sensitivity analysis.

The results of the sensitivity analysis may be used to distinguish model parameters that have a strong influence on the cost function from model parameters that have a weak influence. For example, the first set of model parameters may comprise model parameters that have a strong influence and the second set of model parameters may comprise model parameters that have a weak influence.

The one or more predetermined criteria may comprise at least one of: a determination that a difference in value set of each of the plurality of model parameters between a current iteration and an immediately preceding iteration is below a predetermined threshold; a determination that a difference in the value of the cost function between a current iteration and an immediately preceding iteration is below a predetermined threshold; a determination that a number of iterations has exceeded a predetermined threshold; a determination that an amount of time spent performing the iterative modification exceeds a predetermined amount of time and/or a determination that a difference between each of the plurality of output value sets of the model of the cardiovascular system and the corresponding measured value sets is below an uncertainty of the corresponding measured value set.

Each of these conditions may be used to identify values for the plurality of model parameters for which the cost function may be considered to be minimized.

The processing system may be configured to iteratively modify the plurality of model parameters by: defining a plurality of quantities of interest, wherein a quantity of interest is an output value set of the model of the cardiovascular system corresponding to a predetermined characteristic; identifying measured value sets in the cardiac data that correspond to the quantities of interest; and iteratively modifying the plurality of model parameters of the model to thereby modify a difference between the quantities of interest and the identified measured value sets until the one or more predetermined criteria are met.

The model of the cardiovascular system may be a zero dimensional or one dimensional model. Low dimensional CVS models such as these model cardiac function effectively, while having a relatively small number of model parameters, allowing them to be solved efficiently.

The processing system may be further configured to, following the iterative modification: determine the plurality of output value sets of the model of the cardiovascular system based on the modified value set for each of the plurality of model parameters; and generate one or more cardiac function curves based on the determined plurality of output value sets. Cardiac function curves, such as temporal traces of pressures, volumes and flows, and pressure-volume loops, provide additional clinically useful information.

There is also proposed a computer-implemented method for estimating a left ventricular filling pressure of a subject. The computer-implemented method comprises: obtaining a model of a cardiovascular system that uses a plurality of model parameters to produce output data comprising a plurality of output value sets, each associated with a different non-invasively measurable property of cardiac function, wherein: each output value set contains at least one value of the associated non-invasively measurable property of cardiac function; and the plurality of model parameters includes the target cardiac parameter and/or the output data includes a further output value set for the target cardiac parameter; obtaining cardiac data comprising a plurality of measured value sets, wherein each measured value set is associated with a different output value set and comprises one or more measured values for the non-invasively measurable property associated with the associated output value set; iteratively modifying the values of the plurality of model parameters of the model to thereby modify a difference between the plurality of output value sets of the output data and the corresponding measured value sets in the cardiac data until one or more predetermined criteria are met, wherein the values of the plurality of model parameters of the model are iteratively modified by: dividing the plurality of model parameters into a plurality of sets of one or more model parameters; setting an initial value set for each of the plurality of model parameters; for each set of one or more model parameters in turn, performing iterative steps of: determining one or more output value sets of the model of the cardiovascular system based on a current value set for at least the set of model parameters; calculating a value of a cost function based on the determined one or more output value sets and the measured value sets associated with the one or more output value sets; and adjusting the value set for each model parameter in the set of model parameters based on the calculated value of the cost function, wherein the iterative steps are repeated until a predefined convergence condition is fulfilled; and following the iterative modification, either: if the plurality of model parameters includes the target cardiac parameter, defining the set of one or more values of the modified target cardiac parameter as the estimated set of one or more values for the target cardiac parameter; or if the output data includes the further output value set, defining the further output value set as the estimated set of one or more values for the target cardiac parameter.

Any herein described processing system may be adapted to perform any variation of any herein described method, and vice versa.

There is also proposed a computer program product comprising code means which, when executed on a computer having a processing system, cause the processing system to perform all of the steps of any herein described method.

These and other aspects of the invention will be apparent from and elucidated with reference to the embodiment(s) described hereinafter.

## BRIEF DESCRIPTION OF THE DRAWINGS

For a better understanding of the invention, and to show more clearly how it may be carried into effect, reference will now be made, by way of example only, to the accompanying drawings, in which:

Fig. 1 illustrates a system for estimating a left ventricular filling pressure of a subject, according to an embodiment of the invention;
Fig. 2 illustrates a graphical representation of an example model of the cardiovascular system which may be used in the invention;

Fig. 3 illustrates an example cost function as a weighted sum of areas between curves for each of a plurality of non-invasively measurable indicators;

Fig. 4 illustrates point-wise differences between a measured value and an output value of a CVS model for a cuff pressure at systole and diastole;

Fig. 5 illustrates a method for iteratively determining a value of a cost function and modifying a plurality of model parameters, according to an embodiment of the invention;

Fig. 6 illustrates an alternative method for iteratively determining a value of the cost function CF and modifying the plurality of the model parameters, according to another embodiment of the invention;

Fig. 7 illustrates an example result of a Sobol sensitivity analysis;

Fig. 8 illustrates sub-steps of step 630 of the method of Fig. 6;

Fig. 9 illustrates sub-steps of step 640 of the method of Fig. 6;

Fig. 10 illustrates an alternative method for iteratively determining a value of the cost function CF and modifying the plurality of the model parameters, according to another embodiment of the invention and

Fig. 11 illustrates a computer-implemented method for estimating a left ventricular filling pressure of a subject, according to an embodiment of the invention.

## DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

According to a concept of the invention, there is proposed an approach for determining a set of one or more values for a target cardiac parameter of a subject. A cardiovascular model produces at least one output value set (for a respective at least one property of cardiac function) by processing model parameters. One or more measured value sets, corresponding to non-invasively measurable properties of cardiac function, are processed with corresponding output value sets to modify the model parameters. This modification continues until some predetermined criteria is met, such that the model more closely matches a true representation of the subject's cardiac function. The set of one or more values is then derived from the output of the cardiovascular model and/or from the model parameters of the cardiovascular model.

Embodiments are at least partly based on the realization that a cardiovascular model can be adapted to more closely emulate a subject's true cardiovascular system by modifying the model based on non-invasively measurable data alone. This effectively means that values for certain cardiac parameters, previously only accurately measurable with invasive measurements, can be predicted using a more accurate model than previously available.

Illustrative embodiments may, for example, be employed in any clinical environment in which non-invasive measurements of a cardiac function of a subject can be obtained, e.g. in a clinic, a hospital or even in a subject's home environment.

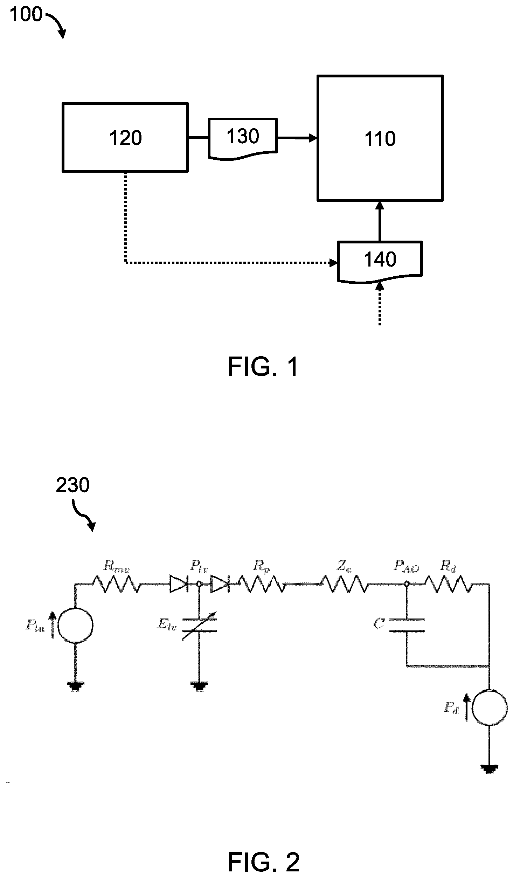

Fig. 1 illustrates a system 100 for estimating a left ventricular filling pressure of a subject, according to an embodiment of the invention. The system 100 comprises a processing system 110 and a memory unit 120. The processing system is, itself, an embodiment of the invention.

The processing system 110 obtains, from the memory unit 120, a model 130 of a cardiovascular system. A model of a cardiovascular system (CVS model) is a biophysical model of (at least parts of) the function of a heart and circulation system. A plurality of model parameters are used to produce output data including a plurality of output value sets. A model parameter is a defined input or intermediate variable or coefficient of the CVS model. Each output value set is associated with a different property of cardiac function, and comprises one or more output values representing a (predicted) value for that property of cardiac function. Models of cardiovascular systems are well known, and suitable CVS models for carrying out the present invention will be apparent to the skilled person. An example of a suitable CVS model is described in more detail below.

More specifically, the CVS model 130 obtained by the processing system 110 is a model of the cardiovascular system that provides output data including output value sets that each represent a different non-invasively measurable property of cardiac function.

The plurality of model parameters of the CVS model includes at least a target cardiac parameter and/or the output data further includes a further output value set for the target cardiac parameter. Thus, a target cardiac parameter may be represented in input, intermediate or output data for the CVS model.

A non-invasively measurable property of cardiac function may be any property that is representative of cardiac function and for which a value may be (preferably directly) determined or derived from non-invasively acquired measurements (e.g. echocardiography measurements, cardiac or cardiovascular MRI, peripheral pressure measurements, etc.). For example, the non-invasively measurable properties may comprise one or more of a left ventricular volume, an aortic pressure, a cuff pressure, an aortic valve flow and/or a mitral valve flow. Approaches for non-invasively measuring these properties have been established in the art.

In some examples, the CVS model 130 may be a low dimensional model of the cardiovascular system (i.e. a zero dimensional or one dimensional CVS model). Low dimensional CVS models effectively model the heart function using a relatively small number of model parameters (i.e. a smaller number than higher dimensional models) and can be solved more efficiently than higher dimensional models. However, the invention is not limited to the use of low dimensional CVS models, and the use of higher dimensional models of the cardiovascular system is also envisaged.

Fig. 2 provides a graphical representation of an example model 230 of the cardiovascular system which may be used in the invention. The CVS model 230 is a simple open-loop zero dimensional model of the left heart. An open-loop CVS model is a CVS model in which the circulation loop is not closed.

In general, a model can define a number of relationships between different possible parameters of the cardiovascular system. These relationships may be representable in formulaic terminology, a number of examples of which are hereafter described.

In example model 230, a left atrial pressure $P_{la}$ is modeled as a constant throughout a cardiac cycle:

$$P_{la}(t) = P_{la} \qquad (1)$$

An inflow $Q_{mv}$ of blood in a left ventricle is modeled as being driven by the difference between the left atrial pressure $P_{la}$ and a left ventricular pressure $P_{lv}$, and a resistance $R_{mv}$ of a mitral valve:

$$Q_{mv} = \frac{P_{la} - P_{lv}}{R_{mv}} \qquad (2)$$

A left ventricular pressure-volume relationship is modeled using an elastance function:

$$P_{lv} = E_{lv}\left(V_{lv} - V_{lv,0}\right) \qquad (3)$$

where $E_{lv}$ is a left ventricular elastance, $V_{lv}$ is a left ventricular volume and $V_{lv,0}$ is a left ventricular dead volume. At an end of diastole (ED), this results in:

$$P_{lv}(t = ED) = P_{la} = LVEDP \qquad (4)$$

where LVEDP is a left ventricular end diastolic filling pressure, which is a left ventricular filling pressure at a time of end diastole.

A change $dV_{lv}/dt$ in left ventricular volume $V_{lv}$ is modeled as a difference between an inflow and an outflow of the left ventricle:

$$\frac{dV_{lv}}{dt} = H_{mv}Q_{mv} - H_{av}Q_{av} \qquad (5)$$

where $H_{mv}$ and $H_{av}$ are states of the mitral valve and an aortic valve respectively (i.e. whether the valve is open or closed), and $Q_{av}$ is an outflow of blood from the left ventricle into an aorta, i.e. an aortic valve flow.

The outflow $Q_{av}$ of blood from the left ventricle into the aorta (i.e. the aortic valve flow) is modeled as being driven by a difference between the left ventricular pressure $P_{lv}$ and an aortic pressure $P_{AO}$, and a resistance $R_p$ of the aortic valve:

$$Q_{av} = \frac{P_{lv} - P_{AO}}{R_p} \qquad (6)$$

The aortic pressure $P_{AO}$ is modeled using a lumped 3-element Windkessel model:

$$\frac{dP_{AO}}{dt} + \frac{1}{R_d C} P_{AO} = \left(1 + \frac{Z_C}{R_d}\right) \frac{Q_{av}}{C} + Z_C Q_{av} \qquad (7)$$

where $Z_c$, $R_d$ and C are respectively a lumped impedance, a resistance and a compliance of an arterial system.

The CVS model 230 can be solved numerically by solving a system of ordinary differential equations. In particular, using a plurality of model parameters that includes the left atrial pressure $P_{la}$ as an input to the model, the model gives an output solution that contains the left ventricular pressure $P_{lv}$, the aortic pressure $P_{AO}$, the left ventricular volume $V_{lv}$, and the inflow $Q_{mv}$ and outflow $Q_{av}$ of blood in the left ventricle (i.e. the flows through the mitral and aortic valves respectively) as functions of time.

This may be more clearly or succulently expressed by introducing a model parameter vector:

$$\mu = [P_{la}, p_1, p_2, \ldots] \qquad (8)$$

where $p_1$, $p_2$, ... are the plurality of model parameters other than the left atrial pressure $P_{la}$. The model solutions for left ventricular volume $V_{lv}$, left ventricular pressure $P_{lv}$, aortic pressure $P_{AO}$, and mitral and aortic flow $Q_{mv}$ and $Q_{av}$ may be expressed as:

$$V_{lv} = V_{lv}(t,\mu), P_{lv} = P_{lv}(t,\mu), P_{AO} = P_{AO}(t,\mu), Q_{mv} = Q_{mv}(t,\mu), Q_{av} = Q_{av}(t,\mu) \qquad (9)$$

In other words, the CVS model 230 may be considered as a mapping of input left atrial pressure $P_{la}$ and other model parameters $p_1$, $p_2$, ... to output cardiovascular function curves (i.e. temporal traces of pressures, volumes and flows). This may also be expressed by assembling the model solutions in a solution vector F (t, $\mu$). The model may be considered as a mapping of the model parameter vector $\mu$ to the solution vector F:

$$\mu \rightarrow F(t,\mu) = [V_{lv}(t,\mu), P_{lv}(t,\mu), P_{AO}(t,\mu), Q_{mv}(t,\mu), Q_{av}(t,\mu)] \qquad (10)$$

It will be appreciated that a number of the elements in the solution vector F represent non-invasively measurable properties of cardiac function. Thus, at least some of the elements of the solution vector are able to represent output value sets that are associated with non-invasively measurable properties of cardiac function. Other elements of the solution vector F represent further output value sets (e.g. representing properties or parameters of cardiac function that have historically only been able to be measured using invasive techniques).

Thus, the value sets in the output data may be effectively split into output data sets (which only represent non-invasively measurable properties of cardiac function) and further output data sets (which are able to represent invasively measurable properties of cardiac function).

One of the parameters represented by a further output value set (if present) may act as the target cardiac parameter for the purposes of disclosed embodiments. For instance, a left ventricular pressure $P_{lv}$ may act as the target cardiac parameter. Other target cardiac parameters may be envisaged for different types or examples of CVS models.

In other examples, the target cardiac parameter is one of the model parameters. In the example CVS model previously described, the left ventricular pressure $P_{lv}$ also acts as a model parameter, as it is used in the calculation of the inflow of blood $Q_{mv}$ and outflow of blood $Q_{av}$, as shown in equations (2) and (6).

Thus, for the example CVS model, the plurality of model parameters includes the target cardiac parameter and the output data includes a further output value set for the target cardiac parameter.

The invention will be described further with reference to this example CVS model 230; however, the invention is not limited to the use of this model, and any suitable model of the cardiovascular system, including closed-loop models, models having more realistic valve behavior, models in which the left atrial pressure is not constant (e.g. models including an atrial elastance function) and models having an extended arterial system, may be used.

Returning to Fig. 1, the processing system 110 obtains cardiac data 140 of a subject. The cardiac data comprises a measured value set for each output value set. Thus, each measured value set is associated with a respective non-invasively measurable property.

The cardiac data 140 may be obtained from the memory unit 120 and/or from a cardiac monitoring system (not shown).

In some examples, the cardiac data 140 may comprise a measured value set for all properties or parameters for which an output value set is provided by the CVS model 130. Of course, the CVS model may provide further output value sets that do not have a counterpart measured value set in the cardiac data 140. In other words, the plurality of non-invasively measurable properties represented by measured value sets in the cardiac data may comprise only a part of the solution of the CVS model. In some examples, each measured value set may comprise a plurality of values (for a particular non-invasively measurable property of cardiac function) measured at different points in the cardiac cycle.

For example, the plurality of non-invasively measurable properties (represented by a respective output value set) may comprise a left ventricular volume and at least one peripheral pressure parameter. It has been identified that, e.g. in the case of the example model 230 described above, measured values of these properties are sufficient to determine an estimate of certain target cardiac parameters such as a left ventricular filling pressure.

In other words, the cardiac data 140 may comprise a measured value set for the left ventricular volume and a measured value set for each of one or more peripheral pressure parameters. The one or more peripheral pressure parameters may comprise a diastolic pressure and/or a systolic pressure.

A left ventricular volume may, for example, be measured by 2D echocardiography (e.g. using the method of discs), 3D echocardiography (e.g. using Philips Dynamic Heart Model), and/or by cardiac MRI. Non-invasive methods of measuring a left ventricular volume will be apparent to the skilled person.

Non-invasive methods of measuring a peripheral pressure will also be apparent to the skilled person. For example, a peripheral pressure may be measured by taking cuff systolic and diastolic pressure measurements (e.g. to measure a brachial pressure, a finger pressure, a neck pressure, a toe pressure, an ankle pressure and/or a leg pressure). In another example, continuous arterial blood pressure measurements may be measured using an ultrasonic device. See, for example, Wang et al. (2018), "Monitoring of the central blood pressure waveform via a conformal ultrasonic device", Nat Biomed Eng, 2:687-695.

It is possible to use one or more transfer functions to convert this peripheral pressures into an approximation of the aortic pressure $P_{AO}$, which converted measurement(s) may form part of the measured value set.

In some examples, the plurality of non-invasively measurable properties (having representative output data sets in the output data) may further comprise an aortic valve flow and/or a mitral valve flow. In other words, the cardiac data 140 may further comprise one or more measured value sets containing at least one aortic valve flow measurement or one or more mitral valve flow measurements.

Non-invasive methods of measuring aortic and/or mitral valve flow will be apparent to the skilled person, and may include cardiovascular MRI flow measurements, Doppler measurements and valve orifice area measurements.

In some examples, the plurality of non-invasively measurable properties (having representative output data sets in the output data) may further comprise a timing of a cardiac cycle event. For example, the cardiac data 140 may further comprise at least one measured value set containing: a timing of an opening of a mitral valve, a timing of a closing of a mitral valve, a timing of an opening of an aortic valve, a timing of a closing of an aortic valve, or a timing of an onset of left ventricular contraction.

Non-invasive methods of measuring a timing of a cardiac cycle event will be apparent to the skilled person. For example, a timing or occurrence of a cardiac cycle event may be determined using ECG data.

Having obtained the CVS model 130 and cardiac data 140, the processing system 110 iteratively modifies the plurality of model parameters of the model to thereby modify a difference between the output value sets of the model and the measured value sets of the cardiac data until one or more predetermined criteria are met. Suitable working examples for the one or more predetermined criteria are described in more detail below.

The processing system then identifies the set of one or more values (i.e. the values at the end of the iterative process) of the target cardiac parameter as the estimated set of one or more values for that target cardiac parameter.

This may comprise identifying the set of one or more values for a further output value set representing the target cardiac parameter (e.g. compared to other output values sets that represent non-invasively measurable properties of cardiac function). This approach can be adopted if the output of the CVS model 130 includes a further output value set for the target cardiac parameter.

Alternatively, this may comprise identifying the set of one or values defined for a model parameter of the CVS model, which is used in the processing performed by the CVS model to produce output data. This approach can be adopted if the model parameters of the CVS model 130 includes the target cardiac parameter, and thereby a set of values for the target cardiac parameter.

In other words, the processing system 110 estimates a set of one or more values for a target cardiac parameter of the subject by fitting the model parameters to the measured values in the cardiac data 140. Put another way, the processing system 110 identifies a set of values of the model parameters for which the solution or output of the model 130 sufficiently, i.e. according to predetermined criteria, corresponds to the subject's cardiac data.

In this way, the obtained cardiac data effectively acts as boundary conditions or boundary values for the CVS model 130.

In some examples, the processing system 110 iteratively modifies the plurality of model parameters by defining a solution vector comprising the plurality of output value sets of the CVS model 130 and a measurement data vector, containing the corresponding measured data sets. The solution vector may be defined in the same way as the solution vector of example model 230 described above: in terms of a model parameter vector $\mu$ containing the model parameters.

The processing system 110 may then define a cost function that quantifies a difference between (part of) the solution vector and the measurement data vector. For example, where the solution vector and measurement data vector are each either calculated or measured at a set of discrete time points $t^k$, the cost function CF may be expressed as:

$$CF(\mu) = \sum_{k=0}^{K} \left( F\left(t^k,\mu\right) - F_d\left(t^k\right) \right)^2 \qquad (11)$$

where $F(t^k, \mu)$ is the solution vector and $F_d(t^k)$ is the measurement data vector.

It will be appreciated that, for the purposes of equation (11), the solution vector used contains only the cardiac properties that are equivalently represented in the measurement data vector. Thus, the cardiac properties defined or represented by the measurement data vector defines the properties that are used in the cost function.

In some embodiments, the cost function is defined using only a subset of the measured value sets in the cardiac data and the corresponding output value sets of the output data and/or using only a selection of values of any of the measured value sets and the corresponding output values in the output value sets.

For instance, quantities of interest (e.g. specific values or value sets) may be identified in the measured value sets and in the corresponding output value sets produced by the CVS model. This quantity of interest may correspond to a particular or predetermined characteristic (e.g. end-diastolic left ventricular volume and/or end-systolic left ventricular volume). A cost function may be defined using only these quantities of interest.

Thus, the cost function may be a function that uses only quantities of interest, i.e. a subset of all possible measured and model-determined parameter values, to define a difference between the solution vector (i.e. the output value sets) and the measurement data vector (i.e. the measured data sets).

The processing system 110 may iteratively determine a value of the cost function CF and modify the plurality of the model parameters based on the value of the cost function until the one or more predetermined criteria are met. The aim is to find the model parameter vector $\mu^*$ (i.e. the values for the model parameters) for which the cost function is minimized.

If the model parameters include the target cardiac parameter, such as a left ventricular pressure, this approach facilitates identification one or more values for the target cardiac parameter from the model parameter vector $\mu^*$.

If the output data produced by the CVS model includes a further output value set for the target cardiac parameter, such as the left ventricular pressure, this approach facilitates identification of one or more values for the target cardiac parameter from the output data (or solution vector) produced by the CVS model.

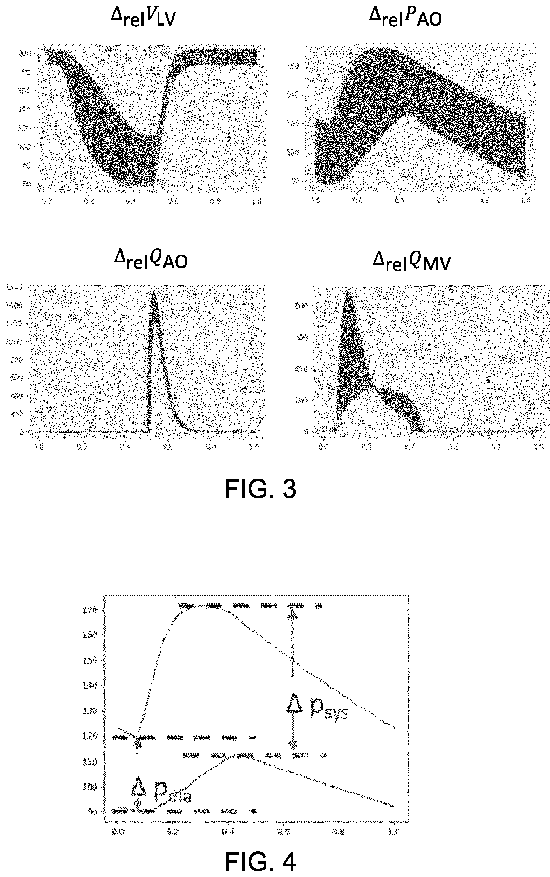

This concept is represented in Fig. 3, which illustrates an example cost function CF as a weighted sum of areas (the shaded parts of Fig. 3) between curves for each of a plurality of non-invasively measurable properties.

In the example shown in Fig. 3, the plurality of non-invasive measurable properties comprises a left ventricular volume $V_{LV}$, an aortic pressure $P_{AO}$, an aortic valve flow $Q_{av}$ and a mitral valve flow $Q_{mv}$. The labels for Fig. 3 identify the associated area illustrated by the accompanying image. The cost function can be expressed as:

$$CF = \Delta_{\mathrm{rel}} V_{\mathrm{LV}} + \Delta_{\mathrm{rel}} P_{\mathrm{AO}} + \Delta_{\mathrm{rel}} Q_{\mathrm{av}} + \Delta_{\mathrm{rel}} Q_{\mathrm{mv}} \qquad (12)$$

The shaded area for each graph represents a difference between a data curve for the non-invasively measurable property (i.e. a curve based on measured values in the cardiac data) and a solution curve (i.e. a curve based on a current output of the CVS model 130). The process of minimizing the example cost function CF corresponds to minimizing a size of the shaded areas.

For some non-invasively measurable properties, there may not be sufficient measured values (in the corresponding measured value set) to construct a data curve, e.g. there may be only one or two measured values for a particular non-invasively measurable property. In such examples, one or more point-wise differences between the measured value(s) and current output value(s) of the CVS model 130 may be used in the cost function instead of areas between curves for one or more of the plurality of non-invasively measurable properties.

For example, Fig. 4 illustrates point-wise differences between a measured value and an output value of the CVS model 130 for a cuff pressure $P_{cuff}$ at systole and diastole ($\Delta P_{sys}$ and $\Delta P_{dia}$ respectively). This may be used instead of the area between aortic pressure curves in the example cost function of Fig. 3 where an aortic pressure data curve is not available. In this case, the cost function could be expressed as:

$$CF = \Delta_{\mathrm{rel}} V_{\mathrm{LV}} + \Delta_{\mathrm{rel}} P_{\mathrm{cuff}} + \Delta_{\mathrm{rel}} Q_{\mathrm{av}} + \Delta_{\mathrm{rel}} Q_{\mathrm{mv}} \qquad (13)$$

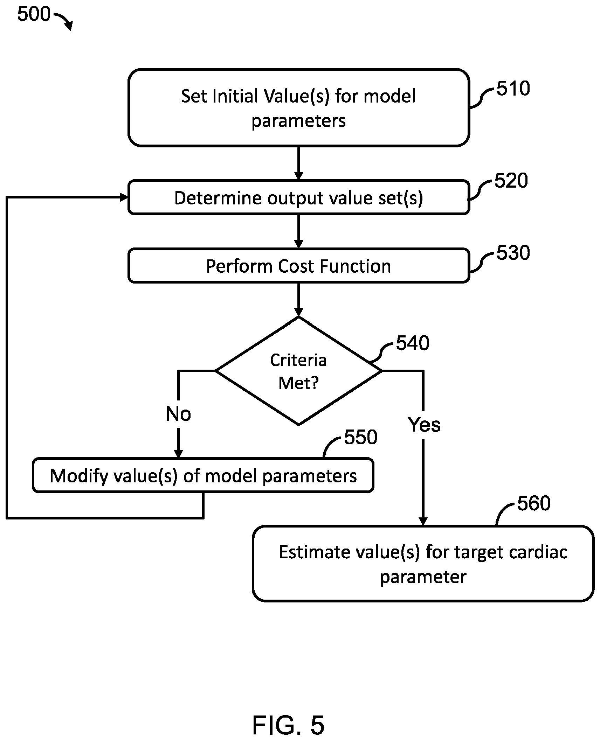

Several methods are envisaged for iteratively determining a value of the cost function CF and modifying the plurality of the model parameters based on the value of the cost function (i.e. in order to minimize the cost function), and further suitable methods will be apparent to the skilled person. In particular, all-at-once optimization methods (e.g. gradient-based minimization methods such as the Broyden-Fletcher-Goldfarb-Shanno (BFGS) algorithm, or non-gradient-based methods such as particle swarm and Bayesian optimization) and multi-level (e.g. bilevel) optimization approaches may be used. Fig. 5 illustrates a method 500 for iteratively determining a value of the cost function CF and modifying the plurality of the model parameters. The method 500 uses an all-at-once approach to minimizing the cost function, in which the cost function is modified as a function of all elements in the model parameter vector $\mu$ (i.e. all of the model parameters) at the same time.

The method 500 begins at step 510. in which an initial value set is set for each of the plurality of model parameters of the CVS model 130. The initial value sets may be randomly assigned values, predefined values or values obtained by user input. For example, the initial values may be defined as the average of their possible ranges. For some model parameters, an initial value may be estimated from the cardiac data 140.

At step 520, the plurality of output value sets of the CVS model 130 are determined based on current value sets for each of the plurality of model parameters. In other words, the CVS model is solved using the current value sets for each of the plurality of model parameters. For a first iteration of the method, the current value sets are the initial value set at step 510. For later iterations, the current value sets are a modified value sets, as described below.

At step 530, a value of the cost function is calculated based on the determined plurality of output value sets and the corresponding measured value sets of the non-invasively measurable properties in the cardiac data 140. In particular examples, a difference between a solution of the CVS model (produced using the current values of the model parameters) and the subject's data is determined.

At step 540, the processing system 110 determines whether the one or more predetermined criteria are met. In response to a determination that the one or more predetermined criteria are not met, the method 500 proceeds to step 550.

At step 550, the value sets for each of the plurality of model parameters is modified based on the calculated value of the cost function. This modification may take place using a gradient descent approach or any other suitable modification approach for minimizing or reducing a cost function, as would be readily apparent to the person skilled in the art.

Steps 520 to 550 are iteratively repeated until it is determined that the one or more predetermined criteria are met. In response to a determination that the one or more predetermined criteria are met, the method 500 proceeds to step 560.

At step 560, the processing system 110 estimates the set of one or more values for the target cardiac parameter of the subject.

Step 560 may be performed, if the plurality of model parameters includes the target cardiac parameter, by identifying the modified value set of the target cardiac parameter (i.e. a current value set of the target cardiac parameter at a most recent iteration) as the set of one or more values for the estimated target cardiac parameter of the subject.

Alternatively, step 560 may be performed, if the output data of the CVS model contains a further output value set for the target cardiac parameter, identifying this further output value set as the set of one or more values for the estimated target cardiac parameter of the subject.

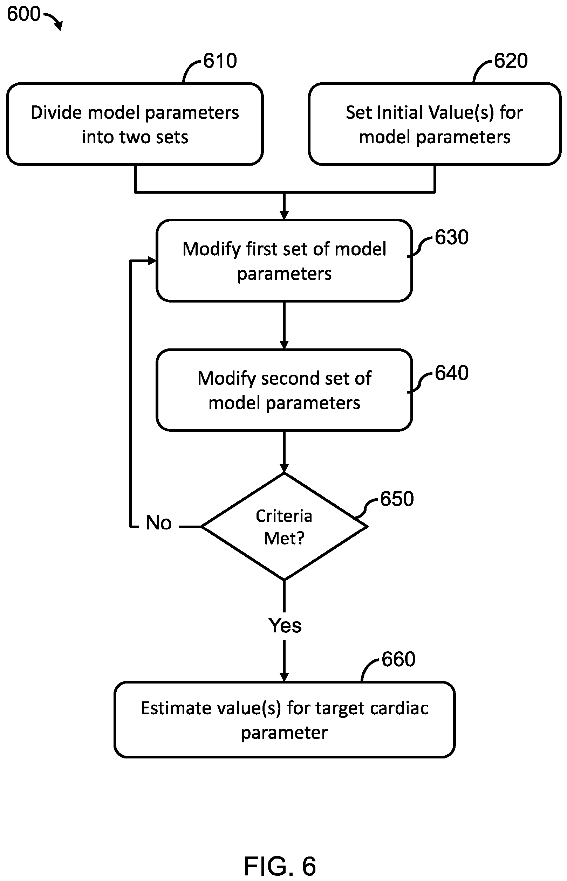

Fig. 6 illustrates an alternative method 600 for iteratively determining a value of the cost function CF and modifying the plurality of the model parameters, according to an embodiment of the invention. The method 600 uses a bilevel optimization approach to minimizing the cost function, in which different groups of parameters are modified independently of one another.

The method 600 begins at step 610, in which the plurality of model parameters is divided into a first set of model parameters and a second set of model parameters.

In some examples, the first set of model parameters may be defined such that the first set comprises only the target cardiac parameter and the second set of model parameters may be defined such that the second set comprises the remaining model parameters (i.e. all of plurality of model parameters except the target cardiac parameter). Of course, in this scenario, the model parameters includes at least the target cardiac parameter. This approach allows the target cardiac parameter to be modified independently of the other model parameters. In other words, the target cardiac parameter may be interpreted as a control input, which is different from the remaining model parameters.

In other examples, the processing system 110 may divide the plurality of model parameters into the first and second sets by performing a sensitivity analysis on the CVS model 130, and dividing the plurality of model parameters into the first set and the second set based on the sensitivity analysis.

Any suitable sensitivity analysis, e.g. the Morris method or the Sobol method, may be used to divide the plurality of model parameters into first and second sets. In some examples, more than one sensitivity analysis may be performed. For instance, the Morris method may be used for a first screening and the Sobol method for a more detailed analysis.

The results of the sensitivity analysis may, for example, be used to distinguish between model parameters that have a stronger influence on the cost function CF and model parameters that have a weaker influence on the cost function. The first set of model parameters may be defined to comprise model parameters having a stronger influence and the second set of model parameters may be defined to comprise model parameters having a weaker influence.

For instance, the first set of model parameters may be defined to comprise model parameters having a Sobol index above a predetermined threshold or to comprise the N model parameters having the highest Sobol indices, where N is a predetermined number. The second set of model parameters may be defined to comprise model parameters having a Sobol index that is not above the predetermined threshold or to comprise all model parameters except the N model parameters having the highest Sobol indices.

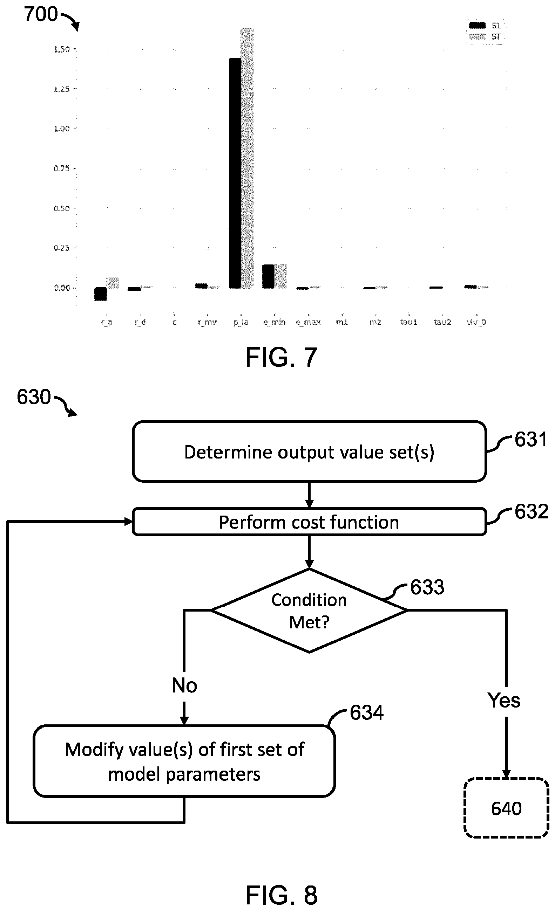

Fig. 7 illustrates an example result 700 of a Sobol sensitivity analysis performed on a CVS model having aortic valve resistance $r_p$, arterial resistance $r_d$, arterial impedance c, mitral valve resistance $r_{mv}$, left atrial pressure $P_{la}$, elastance $e_{min}$, elastance $e_{max}$, $m_1$, $m_2$, $\tau_1$, $\tau_2$, and left ventricular dead volume $V_{lv,0}$ as the model parameters.

In example result 700, the left atrial pressure $P_{la}$ is the dominant model parameter, having the strongest effect on the cost function (S1) and the most significant interactions with the other model parameters (ST), followed by the elastance $e_{min}$ and the aortic valve resistance $r_p$. The Sobol indices of the remaining model parameters are very small.

The model parameters may be divided into first and second sets based on the example result 700 by, for example, assigning the left atrial pressure $P_{la}$ to the first set and the remaining model parameters to the second set, or by assigning the left atrial pressure $P_{la}$, elastance $e_{min}$ and aortic valve resistance $r_p$ to the first set and the remaining model parameters to the second set. In another example, the model parameters may be divided into three sets: a first set comprising only the left atrial pressure, a second set comprising the elastance and the aortic valve resistance, and a third set comprising the remaining model parameters. The skilled person would be readily capable of adapting the method 600 to a method having three sets of model parameters.

Returning to Fig. 6, at step 620, initial values are set or defined for each of the plurality of model parameters. The initial values may be randomly assigned values, predefined values or values obtained by user input.

In some examples, in step 620 at least some of the initial values may be estimated from the available measurements (e.g. from the measurement data set). This approach may exploit existing statistical models for defining or predicting parameter values from non-invasively measurable data (which may not be sufficiently accurate for a specific subject, but may prove a good starting point for tuning the model parameters to the specific subject).

At step 630, the processing system 110 iteratively modifies the value set for each of the first set of the plurality of model parameters until a first predefined convergence condition is fulfilled. Step 630 will be described in greater detail with reference to Fig. 8, which illustrates the sub-steps of step 630.

At sub-step 631, the plurality of output value sets of the CVS model 130 are determined based on a current value for each of the plurality of model parameters.

At sub-step 632, a value of the cost function is calculated based on the determined plurality of output values.

At sub-step 633, the processing system determines whether the first predefined convergence condition is fulfilled. This may, for instance, be fulfilled when the value of the cost function falls below some first predetermined threshold. Other examples are provided later in this disclosure.

In response to a determination that the first predefined convergence condition is not fulfilled, step 630 proceeds to sub-step 634.

At sub-step 634, the value set for each of the first set of model parameters is adjusted based on the calculated value of the cost function. The value sets of the model parameters in the second set are not adjusted during sub-step 634.

Sub-steps 631 to 634 are iteratively repeated until it is determined that the first predefined convergence condition is fulfilled. In response to a determination that the first predefined convergence condition is fulfilled, the method 600 proceeds to step 640.

Returning to Fig. 6, at step 640, the processing system 110 iteratively modifies the value set for each of the second set of the plurality of model parameters until a second predefined convergence condition is fulfilled.

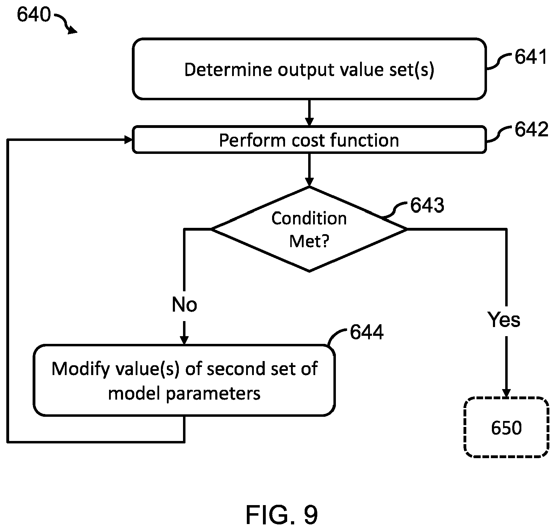

Step 640 will be described in greater detail with reference to Fig. 9, which illustrates the sub-steps of step 640.

At sub-step 641, the plurality of output value sets of the CVS model 130 are determined based on a current value set for each of the plurality of model parameters.

At sub-step 642, a value of the cost function is calculated based on the determined plurality of output value sets (and the measured value sets).

At sub-step 643, the processing system determines whether the second predefined convergence condition is fulfilled. This may, for instance, be fulfilled when the value of the cost function falls below some second predetermined threshold. Other examples are provided later in this disclosure.

In response to a determination that the second predefined convergence condition is not fulfilled, step 640 proceeds to sub-step 644.

At sub-step 644, the value set for each of the second set of model parameters is adjusted based on the calculated value of the cost function. The value sets of any model parameters in the first set are not adjusted during sub-step 644.

Sub-steps 641 to 644 are iteratively repeated until it is determined that the second predefined convergence condition is fulfilled. In response to a determination that the second predefined convergence condition is fulfilled, the method 600 proceeds to step 650.

Steps 630 and 640 may be better understood by considering the step 610 of dividing the model parameters into first and second sets in terms of splitting the model parameter vector $\mu$ into two components. For example, where the plurality of model parameters is divided according to the results of a sensitivity analysis, the model parameter vector $\mu$ may be expressed as being split into two components:

$$\mu = \left[\mu^{\text{strong}}, \mu^{\text{weak}}\right] \qquad (14)$$

where $\mu^{\text{strong}}$ is a vector containing the first set of model parameters (i.e. the model parameters having a stronger influence), and $\mu^{\text{weak}}$ is a vector containing the second set of model parameters (i.e. the model parameters having a weaker influence).

At step 630, the aim is to find the vector $\mu^{\text{strong*}}$, where:

$$\mu^{\text{strong*}} = \operatorname{argmin} CF\left(\mu^{\text{strong}}, \mu^{\text{weak}}\right) \qquad (15)$$

At step 640, the aim is to find the vector $\mu^{\text{weak*}}$, where:

$$\mu^{\text{weak*}} = \operatorname{argmin} CF\left(\mu^{\text{strong*}}, \mu^{\text{weak}}\right) \qquad (16)$$

At step 650, the processing system 110 determines whether the one or more predetermined criteria are met.

In response to a determination that the one or more predetermined criteria are not met, steps 630 to 650 are iteratively repeated until it is determined that the one or more predetermined criteria are met. In some examples, the predetermined criteria may be met if both the first and second convergence condition are met and upheld.

In response to a determination that the one or more determined criteria are met, the method 600 proceeds to step 660.

At step 660, the processing system 110 estimates the target cardiac parameter of the subject by identifying the modified value set of the target cardiac parameter model parameter as the estimated target cardiac parameter of the subject.

The embodiments described with reference to Figs. 6 to 9 effectively divide the model parameters into two sets, and perform an iterative modification process on each set of model parameters. This approach can be expanded to comprise dividing the model parameters into N sets (N>1), and performing an iterative modification is performed on each set of parameters

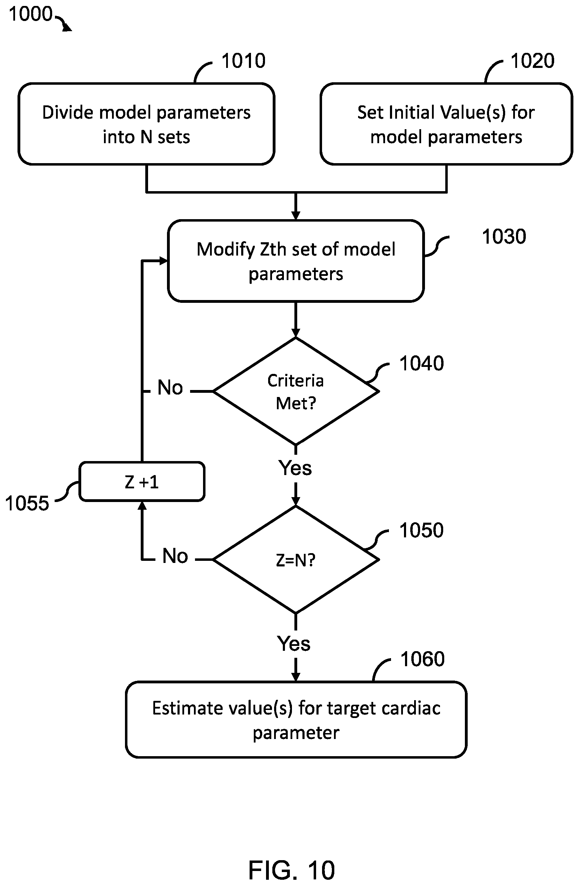

This generalized approach is illustrated by Fig. 10, which illustrates a method 1000 for iteratively determining a value of the cost function CF and modifying the plurality of the model parameters.

The method 1000 comprises a step 1010 of dividing the model parameters into a plurality of N sets, where N is any positive integer greater than 1. The method 1000 also comprises a step 1020 of setting one or more initial value(s) for model parameters.

Each set of model parameters is iteratively modified in turn until some predetermined (termination) criteria is met for each instance of the iterative modification. This is illustrated through steps 1030 to 1050 of Fig. 10.

In step 1030, the Zth set of parameters (where Z is initially 1) is modified.

In step 1040, the method determines whether some predetermined criteria has been met, e.g. whether a cost function for the Zth set of parameters meets some predetermined criteria. The cost function may differ for each set of parameters. Of course, a same cost function may be shared between two or more sets of parameters.

If the outcome of step 1040 is negative, the method reverts back to step 1030. If the outcome of step 1040 is positive, the method determines (in step 1050) whether all sets have been processed. If all sets have been processed, the method moves to a step 1060 of estimating the value(s) for the target cardiac parameter. Otherwise, step 1030 is performed for the next set of parameters (e.g. by adding 1 to the value of Z in a step 1055 and then reverting back to step 1030).

In some examples, rather than moving to step 1060 if all sets have been processed, the method may revert back to step 1030 for the first set of model parameters that was processed. Thus, Z may effectively be reset to 1. This is because the modifications to the subsequent sets may have affected the accuracy of the model. This repeating of modifying the sets of model parameters may be performed until some predetermined criteria has been met, examples of which will be later described.

Suitable approaches for modifying parameters have been previously described.

The division of the model parameters into N sets may be performed, for instance, based on a sensitivity analysis of the model parameters.

One particularly advantageous approach for sets of model parameters for the example model described with reference to Fig. 2 is hereafter described.

This proposed approach combines assumptions based on an understanding of the physiology and cardiac function, results of sensitivity analysis of the example model of the CV system, and relations and interactions between the model parameters. The hereafter described approach may thereby generate model parameters that more accurately or correctly represent the true processes taking place in the subject.

In this approach, a first set of model parameters includes the unknown parameters $Z_c$, $R_d$ and C, which are respectively a lumped impedance, a resistance and a compliance of an arterial system (see equation (7)). These three parameters can be referred to as the Windkessel (WK) parameters, as they form part of the Windkessel model.

In this approach, a second set of model parameters includes only the filling pressure $P_{la}$ and the left ventricular dead volume $V_{lv,0}$. This second set has been identified, from a sensitivity analysis, as being particularly effective in modifying the solution vector produced by the example model.

In this approach, a third set of model parameters includes all remaining parameters of the example model. The third set can effectively fine tune the example model based on the coarser modifications performed using the first and second sets.

As previously explained, the cost function used in iteratively modifying each set of model parameters may differ for the set of model parameters being processed.

As an example, for the first set of model parameters, the cost function may be a difference between a predicted aortic pressure (e.g. in the solution vector or output data and/or by processing aortic flow $Q_{av}$ using equation (7)) and a true aortic pressure $P_{AO}$ (in the cardiac data or measurement data vector). The aortic flow $Q_{av}$ may be available in the cardiac data or may be derived from another parameter available in the measurement data vector.

For the second and third set of model parameters, the cost function may be a quantified difference (e.g. summed error, mean error or mean squared error) between all model parameters shared by the solution vector and the measurement data vector. Other suitable examples would be apparent.

For processing the first set of model parameters, in the case where the aortic flow $Q_{av}$ is not available in the measurement data vector, e.g. when operating under the assumption that there is no regurgitation through the mitral valve during contraction, it is possible to estimate the aortic flow $Q_{av}$ as the change in the LV volume in time, such that Qav(t) = dVlv(t)/dt.

In some examples, the measurement data vector contains a peripheral pressure measurement, such as a brachial cuff measurement $P_{curr}$. It is possible to use one or more transfer functions to convert this measurement into an approximation of the aortic pressure $P_{AO}$. Suitable transfer functions are readily apparent to the skilled person.

The various iterative modification processes performed in such an iterative process can be carried out using any known modification process, such as common gradient-based minimization methods, no-gradient based and global optimization methods (particle swarm, genetic algorithms, Bayesian optimization, etc.).

In one example, an iterative modification process may comprise generating an output value set for each of a plurality of different value sets for the set of model parameters that are being modified, determining a cost function for each output value set and selecting the values for the set of model parameter associated with the lowest cost function. The plurality of different values may, for instance, comprise a plurality of value sets - each value set representing a value for each of the plurality of parameters to be modified. In some examples, there may be a maximum and minimum value for each parameter, which could be defined based on known ranges for the parameter. Each value set may represent a sample within these maximum and minimum bounds. The plurality of value sets does not need to contain all possible values for each parameter, rather, a sample selection of the possible values for each parameter may be used.

In any above-described embodiment, it is possible to define one or more bounds or constraints for one or more model parameters by processing determined values for other model parameters. In particular, the values for model parameters that are part of subsequent sets of model parameters to earlier processed model parameters may be bound (at an upper and/or lower bound) based on determined values for model parameters determined in earlier processing. In this context, a subsequent set is a set for which model parameters have

Consider a scenario in which a certain set of model parameters includes the filling pressure $P_{la}$ and the left ventricular dead volume $V_{lv,0}$ and a later set of model parameters (i.e. a set of model parameters that is modified after the certain set) contains a left ventricular elastance Elv.

In this scenario, it is possible to define a constraint $E_{min}$ for the left ventricular elastance Elv using the following equation:

$$E_{min} = \frac{P_{la}}{EDV - V_{lv,0}} \qquad (17)$$

EDV is the end-diastolic volume, which can be identified in the cardiac data.

It is possible to define the left ventricular elastance Elv (used in equation (3) using the following equation:

$$E_{lv} = (E_{max} - E_{min}) * \hat{E} + E_{min} \qquad (18)$$

where $\hat{E}(t)$ is a normalized elastance function that can be represented in different formulations. A suitable example is the so-called Double-Hill representation, but others will be apparent to the skilled person, and can employ combinations of sine- and exponential function. $E_{min}$ and $E_{max}$ thereby represent constraints for the left ventricular elastance $E_{lv}$.

This example illustrates how it is possible to set a constraint for a parameter based on one or more earlier determined values for one or more other model parameters.

As previously mentioned, the processing system iteratively modifies the plurality of model parameters to thereby modify a difference between the output values and the measured values until one or more predefined criteria or convergence condition(s) are/is met. Termination criteria for optimization processes are well known, and suitable criteria to use as the one or more predefined criteria will be apparent to the skilled person.

For example, the one or more predetermined criteria or convergence condition may comprise at least one of: a determination that a difference in the value of each of the plurality of model parameters between a current iteration and an immediately preceding iteration is below a predetermined threshold; a determination that a difference in the value of the cost function between a current iteration and an immediately preceding iteration is below a predetermined threshold; a determination that a number of iterations has exceeded a predetermined threshold; and/or a determination that a difference between each of the plurality of output values of the CVS model 130 and the corresponding measured value in the cardiac data 140 is below an uncertainty of the measured value. Suitable values for the predetermined thresholds will be apparent to the skilled person. In some examples, the one or more predetermined criteria may comprise at least one criterion relating to a history of the model parameter values, in order to terminate the process when the solution is oscillating between two values.

Where the one or more predetermined criteria or convergence condition comprises several predetermined criteria, the processing system may iteratively modify the model parameters until all of the predetermined criteria are met or until at least one of the predetermined criteria has been met.

In some examples, the output data of the CVS model 130 may be used to generate cardiac function curves for the subject. The processing system 110 may determine the plurality of output value sets (and optionally, if present, any further output value sets) of the CVS model based on the modified value set for each of the model parameters (i.e. the value sets at the end of the iteration process, once the one or more predetermined criteria are met), and generate one or more cardiac function curves based on the determined output value sets and (if present) any further output value sets.

The one or more cardiac function curves may include one or more of: a temporal trace of a pressure (e.g. a left ventricular and/or an aortic pressure) over one or more cardiac cycles, a temporal trace of a volume (e.g. a left ventricular volume) over one or more cardiac cycles, a temporal trace of a flow (e.g. a flow through a mitral and/or aortic valve) over one or more cardiac cycles and/or a pressure-volume loop.

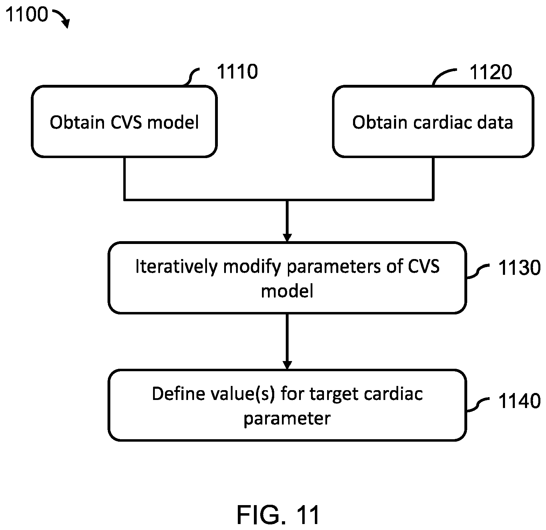

Fig. 11 illustrates a computer-implemented method 1100 for estimating a target cardiac parameter of a subject.

The method 1100 begins at step 1110, in which a model of a cardiovascular system is obtained. The model of the cardiovascular system uses a plurality of model parameters to produce output data comprising a plurality of output value sets, each associated with a different non-invasively measurable property of cardiac function. Each output value set contains at least one value representing a value of the associated non-invasively measurable property of cardiac function. The plurality of model parameters includes the target cardiac parameter and/or the output data includes a further output value set for the target cardiac parameter

At step 1120, cardiac data for the subject is obtained. The cardiac data comprises a plurality of measured value sets, wherein each measured value set is associated with a different output value set and comprises one or more measured values for the non-invasively measurable property associated with the associated output value set.

At step 1130, the method iteratively modifies the value sets of the plurality of model parameters of the CVS to thereby modify a difference between the plurality of output value sets of the output data and the corresponding measured value sets in the cardiac data until one or more predetermined criteria are met.

Step 1140 is performed following the iterative modification, and establishes or defines the set of one or more values for the target cardiac parameter. Step 1140 comprises if the plurality of model parameters includes the target cardiac parameter, defining the set of one or more values of the modified target cardiac parameter as the estimated set of one or more values for the target cardiac parameter. Alternatively, step 1140 may comprise, if the output data includes the further output value set, define the further output value set as the estimated set of one or more values for the target cardiac parameter.

The method 1100 may further comprise a step 1150 of providing, at an output interface, a user-perceptible output responsive to the estimated set of one or more values of the target cardiac parameter. The user-perceptible output may be a visual representation of the estimated set (e.g. in the form of a curve or display of any values).

It will be understood that the disclosed methods are computer-implemented methods. As such, there is also proposed a concept of a computer program comprising code means for implementing any described method when said program is run on a processing system.

The skilled person would be readily capable of developing a processor for carrying out any herein described method. Thus, each step of a flow chart may represent a different action performed by a processor, and may be performed by a respective module of the processor.

As discussed above, the system makes use of a processor to perform the data processing. The processor can be implemented in numerous ways, with software and/or hardware, to perform the various functions required. The processor typically employs one or more microprocessors that may be programmed using software (e.g. microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

**Claims**

1. A processing system (110) for estimating a set of one or more values for a target cardiac parameter of a subject, the processing system being configured to:

   obtain a model (130) of a cardiovascular system that uses a plurality of model parameters to produce output data comprising a plurality of output value sets, each associated with a different non-invasively measurable property of cardiac function, wherein:

   each output value set contains at least one value of the associated non-invasively measurable property of cardiac function; and
   the plurality of model parameters includes the target cardiac parameter and/or the output data includes a further output value set for the target cardiac parameter;

   obtain cardiac data (140) comprising a plurality of measured value sets, wherein each measured value set is associated with a different output value set and comprises one or more measured values for the non-invasively measurable property associated with the associated output value set;
   iteratively modify the values of the plurality of model parameters of the model to thereby modify a difference between the plurality of output value sets of the output data and the corresponding measured value sets in the cardiac data until one or more predetermined criteria are met, wherein the processing system is configured to iteratively modify the values of the plurality of model parameters of the model by:

   dividing the plurality of model parameters into a plurality of sets of one or more model parameters;
   setting an initial value set for each of the plurality of model parameters;
   for each set of one or more model parameters in turn, performing iterative steps of:

   determining one or more output value sets of the model of the cardiovascular system based on a current value set for at least the set of model parameters;
   calculating a value of a cost function based on the determined one or more output value sets and the measured value sets associated with the one or more output value sets; and
   adjusting the value set for each model parameter in the set of model parameters based on the calculated value of the cost function,

   wherein the iterative steps are repeated until a predefined convergence condition is fulfilled; and

following the iterative modification, either:

> if the plurality of model parameters includes the target cardiac parameter, define the set of one or more values of the modified target cardiac parameter as the estimated set of one or more values for the target cardiac parameter; or
> if the output data includes the further output value set for the target cardiac parameter, define the further output value set as the estimated set of one or more values for the target cardiac parameter.

2. The processing system (110) of claim 1, wherein the target cardiac parameter is a left ventricular pressure.

3. The processing system (110) of claim 1 or 2, wherein the plurality of non-invasively measurable properties comprises a left ventricular volume and at least one peripheral pressure property .

4. The processing system (110) of any of claims 1 to 3, wherein the plurality of non-invasively measurable indicators comprises at least one of: an aortic valve flow, a mitral valve flow and/or a timing of a cardiac cycle event.

5. The processing system (110) of any of claims 1 to 4, wherein the processing system is configured to:

> divide the plurality of model parameters into a plurality of sets of one or more model parameters by dividing the plurality of model parameters into a first set of model parameters and a second set of model parameters;
> modify a value set for each of the first set of model parameters by:
>
>> determining the plurality of output value sets of the model (130) of the cardiovascular system based on a current value set for each of the plurality of model parameters;
>> calculating a value of the cost function based on the determined plurality of output value sets;
>> adjusting the value set for each of the first set of model parameters based on the calculated value of the cost function; and
>> iteratively repeating the steps of determining the plurality of output value sets, calculating the value of the cost function, and adjusting the value set for each of the first set of model parameters until a first predefined convergence condition is fulfilled;
>
> modify a value set for each of the second set of model parameters by:
>
>> determining the plurality of output value sets of the model of the cardiovascular system based on a current value set for each of the plurality of model parameters;
>> calculating a value of the cost function based on the determined plurality of output value sets;
>> adjusting the value set for each of the second set of model parameters based on the calculated value of the cost function; and
>> iteratively repeating the steps of determining the plurality of output value sets, calculating the value of the cost function, and adjusting the value set for each of the second set of model parameters until a second predefined convergence condition is fulfilled; and
>> iteratively repeat the steps of modifying a value set for each of the first set of model parameters and modifying a value set for each of the second set of model parameters until the one or more predetermined criteria are met.

6. The processing system (110) of claim 5, wherein the plurality of model parameters includes the target cardiac parameter and the processing system is configured to divide the plurality of model parameters into a first set of model parameters and a second set of model parameters by:

> defining the first set of model parameters to comprise only the target cardiac parameter; and
> defining the second set of model parameters to comprise the remaining model parameters.

7. The processing system (110) of any of claims 1 to 6, wherein the processing system is configured to divide the plurality of model parameters into a plurality of sets of one or more model parameters:

> performing a sensitivity analysis on the model of the cardiovascular system; and
> dividing the plurality of model parameters into the plurality of sets of one or more model parameters based on the sensitivity analysis.

8. The processing system (110) of any of claims 1 to 7, wherein the one or more predetermined criteria comprises at least one of: a determination that a difference in value set of each of the plurality of model parameters between a current iteration and an immediately preceding iteration is below a predetermined threshold; a determination that a difference in the value of the cost function between a current iteration and an immediately preceding iteration is below a predetermined threshold; a determination that a number of iterations has exceeded a predetermined threshold; a determination that an amount of time spent performing the iterative modification exceeds a predetermined amount of time and/or a determination that a difference between each of the plurality of output value sets of the model (130) of the cardiovascular system and the corresponding measured value sets is below an uncertainty of the corresponding measured value set.

9. The processing system (110) of any of claims 1 to 8, wherein the processing system is further configured to, following the iterative modification:

   determine the plurality of output value sets of the model (130) of the cardiovascular system based on the modified value set for each of the plurality of model parameters; and
   generate one or more cardiac function curves based on the determined plurality of output value sets.

10. A computer-implemented method (1100) for estimating a left ventricular filling pressure of a subject, the computer-implemented method comprising:

   obtaining (1110) a model (130) of a cardiovascular system that uses a plurality of model parameters to produce output data comprising a plurality of output value sets, each associated with a different non-invasively measurable property of cardiac function, wherein:

      each output value set contains at least one value of the associated non-invasively measurable property of cardiac function; and
      the plurality of model parameters includes the target cardiac parameter and/or the output data includes a further output value set for the target cardiac parameter;

   obtaining (1120) cardiac data (140) comprising a plurality of measured value sets,
   wherein each measured value set is associated with a different output value set and comprises one or more measured values for the non-invasively measurable property associated with the associated output value set;
   iteratively modifying (1130) the values of the plurality of model parameters of the model to thereby modify a difference between the plurality of output value sets of the output data and the corresponding measured value sets in the cardiac data until one or more predetermined criteria are met, wherein the values of the plurality of model parameters of the model are iteratively modified by:

      dividing the plurality of model parameters into a plurality of sets of one or more model parameters;
      setting an initial value set for each of the plurality of model parameters;
      for each set of one or more model parameters in turn, performing iterative steps of:

         determining one or more output value sets of the model of the cardiovascular system based on a current value set for at least the set of model parameters;
         calculating a value of a cost function based on the determined one or more output value sets and the measured value sets associated with the one or more output value sets; and
         adjusting the value set for each model parameter in the set of model parameters based on the calculated value of the cost function,

      wherein the iterative steps are repeated until a predefined convergence condition is fulfilled; and

   following the iterative modification, either (1140):

      if the plurality of model parameters includes the target cardiac parameter, defining the set of one or more values of the modified target cardiac parameter as the estimated set of one or more values for the target cardiac parameter; or
      if the output data includes the further output value set for the target cardiac parameter, defining the further output value set as the estimated set of one or more values for the target cardiac parameter.

11. A computer program product comprising code means which, when executed on a computer having a processing system, cause the processing system to perform all of the steps of the method (1100) according to claim 10.

**Patentansprüche**

1. Verarbeitungssystem (110) zum Schätzen eines Satzes eines oder mehrerer Werte für einen Ziel-Herzparameter eines Subjekts, wobei das Verarbeitungssystem dazu konfiguriert ist:

    ein Modell (130) eines Herz-Kreislauf-Systems zu erhalten, das eine Vielzahl von Modellparametern verwendet, um Ausgabedaten zu erzeugen, die eine Vielzahl von Ausgabewertsätzen umfassen, die jeweils einer anderen nichtinvasiv messbaren Eigenschaft der Herzfunktion zugeordnet sind, wobei:

    jeder Ausgabewertsatz mindestens einen Wert der zugeordneten nichtinvasiv messbaren Eigenschaft der Herzfunktion enthält; und
    die Vielzahl von Modellparametern den Ziel-Herzparameter beinhaltet und/oder die Ausgabedaten einen weiteren Ausgabewertsatz für den Ziel-Herzparameter beinhalten;

    Herzdaten (140) zu erhalten, die eine Vielzahl von Messwertsätzen umfassen, wobei jeder Messwertsatz einem anderen Ausgabewertsatz zugeordnet ist und einen oder mehrere Messwerte für die nichtinvasiv messbare Eigenschaft, die dem zugeordneten Ausgabewertsatz zugeordnet ist, umfasst;
    die Werte der Vielzahl von Modellparametern des Modells iterativ zu modifizieren, um dadurch eine Differenz zwischen der Vielzahl von Ausgabewertsätzen der Ausgabedaten und den entsprechenden Messwertsätzen in den Herzdaten zu modifizieren, bis ein oder mehrere vorbestimmte Kriterien erfüllt sind, wobei das Verarbeitungssystem dazu konfiguriert ist, die Werte der Vielzahl von Modellparametern des Modells iterativ zu modifizieren durch:

    Aufteilen der Vielzahl von Modellparametern in eine Vielzahl von Sätzen eines oder mehrerer Modellparameter;
    Festlegen eines Anfangswertsatzes für jeden der Vielzahl von Modellparametern;
    Durchführen der folgenden iterativen Schritte für jeden Satz eines oder mehrerer Modellparameter nacheinander:

    Bestimmen eines oder mehrerer Ausgabewertsätze des Modells des Herz-Kreislauf-Systems auf Basis eines aktuellen Wertsatzes für mindestens den Satz Modellparameter;
    Berechnen eines Werts einer Kostenfunktion auf Basis des einen oder der mehreren bestimmten Ausgabewertsätze und der Messwertsätze, die dem einen oder den mehreren Ausgabewertsätzen zugeordnet sind; und
    Anpassen des Wertsatzes für jeden Modellparameter in dem Satz Modellparameter auf Basis des berechneten Werts der Kostenfunktion,

    wobei die iterativen Schritte wiederholt werden, bis eine vordefinierte Konvergenzbedingung erfüllt ist; und

    nach der iterativen Modifikation, entweder:

    wenn die Vielzahl von Modellparametern den Ziel-Herzparameter beinhaltet, den Satz eines oder mehrerer Werte des modifizierten Ziel-Herzparameters als den geschätzten Satz eines oder mehrerer Werte für den Ziel-Herzparameter zu definieren; oder
    wenn die Ausgabedaten den weiteren Ausgabewertsatz für den Ziel-Herzparameter beinhalten, den weiteren Ausgabewertsatz als den geschätzten Satz eines oder mehrerer Werte für den Ziel-Herzparameter zu definieren.

2. Verarbeitungssystem (110) nach Anspruch 1, wobei der Ziel-Herzparameter ein linksventrikulärer Druck ist.

3. Verarbeitungssystem (110) nach Anspruch 1 oder 2, wobei die Vielzahl von nichtinvasiv messbaren Eigenschaften ein linksventrikuläres Volumen und mindestens eine periphere Druckeigenschaft umfasst.

4. Verarbeitungssystem (110) nach einem der Ansprüche 1 bis 3, wobei die Vielzahl von nichtinvasiv messbaren Indikatoren mindestens eines umfasst von: einem Aortenklappenfluss, einem Mitralklappenfluss und/oder einem Zeitpunkt eines Herzzyklusereignisses.

5. Verarbeitungssystem (110) nach einem der Ansprüche 1 bis 4, wobei das Verarbeitungssystem dazu konfiguriert ist:

die Vielzahl von Modellparametern in eine Vielzahl von Sätzen eines oder mehrerer Modellparameter aufzuteilen durch Aufteilen der Vielzahl von Modellparametern in einen ersten Satz Modellparameter und einen zweiten Satz Modellparameter;
einen Wertsatz für jeden des ersten Satzes Modellparameter zu modifizieren durch:

Bestimmen der Vielzahl von Ausgabewertsätzen des Modells (130) des Herz-Kreislauf-Systems auf Basis eines aktuellen Wertsatzes für jeden der Vielzahl von Modellparametern;
Berechnen eines Werts der Kostenfunktion auf Basis der bestimmten Vielzahl von Ausgabewertsätzen;
Anpassen des Wertsatzes für jeden des ersten Satzes Modellparameter auf Basis des berechneten Werts der Kostenfunktion; und
iteratives Wiederholen der Schritte des Bestimmens der Vielzahl von Ausgabewertsätzen, Berechnens des Werts der Kostenfunktion und Anpassens des Wertsatzes für jeden des ersten Satzes Modellparameter, bis eine erste vordefinierte Konvergenzbedingung erfüllt ist;

einen Wertsatz für jeden des zweiten Satzes Modellparameter zu modifizieren durch:

Bestimmen der Vielzahl von Ausgabewertsätzen des Modells des Herz-Kreislauf-Systems auf Basis eines aktuellen Wertsatzes für jeden der Vielzahl von Modellparametern;
Berechnen eines Werts der Kostenfunktion auf Basis der bestimmten Vielzahl von Ausgabewertsätzen;
Anpassen des Wertsatzes für jeden des zweiten Satzes Modellparameter auf Basis des berechneten Werts der Kostenfunktion; und
iteratives Wiederholen der Schritte des Bestimmens der Vielzahl von Ausgabewertsätzen, Berechnens des Werts der Kostenfunktion und Anpassens des Wertsatzes für jeden des zweiten Satzes Modellparameter, bis eine zweite vordefinierte Konvergenzbedingung erfüllt ist; und
iteratives Wiederholen der Schritte des Modifizierens eines Wertsatzes für jeden des ersten Satzes Modellparameter und Modifizierens eines Wertsatzes für jeden des zweiten Satzes Modellparameter, bis das eine oder die mehreren vorbestimmten Kriterien erfüllt sind.

6. Verarbeitungssystem (110) nach Anspruch 5, wobei die Vielzahl von Modellparametern den Ziel-Herzparameter beinhaltet und das Verarbeitungssystem dazu konfiguriert ist, die Vielzahl von Modellparametern in einen ersten Satz Modellparameter und einen zweiten Satz Modellparameter aufzuteilen durch:

Definieren des ersten Satzes Modellparameter so, dass er nur den Ziel-Herzparameter umfasst; und
Definieren des zweiten Satzes Modellparameter so, dass er die restlichen Modellparameter umfasst.

7. Verarbeitungssystem (110) nach einem der Ansprüche 1 bis 6, wobei das Verarbeitungssystem dazu konfiguriert ist, die Vielzahl von Modellparametern in eine Vielzahl von Sätzen eines oder mehrerer Modellparameter aufzuteilen durch:

Durchführen einer Sensitivitätsanalyse am Modell des Herz-Kreislauf-Systems; und
Aufteilen der Vielzahl von Modellparametern in die Vielzahl von Sätzen eines oder mehrerer Modellparameter auf Basis der Sensitivitätsanalyse.

8. Verarbeitungssystem (110) nach einem der Ansprüche 1 bis 7, wobei das eine oder die mehreren vorbestimmten Kriterien mindestens eines umfassen von: einer Bestimmung, dass eine Differenz im Wertsatz jedes der Vielzahl von Modellparametern zwischen einer aktuellen Iteration und einer unmittelbar vorhergehenden Iteration unter einer vorbestimmten Schwelle liegt; einer Bestimmung, dass eine Differenz im Wert der Kostenfunktion zwischen einer aktuellen Iteration und einer unmittelbar vorhergehenden Iteration unter einer vorbestimmten Schwelle liegt; einer Bestimmung, dass eine Anzahl von Iterationen eine vorbestimmte Schwellen überschritten hat; einer Bestimmung, dass eine für die Durchführung der iterativen Modifikation aufgewendete Zeitdauer eine vorbestimmte Zeitdauer überschreitet und/oder einer Bestimmung, dass eine Differenz zwischen jedem der Vielzahl von Ausgabewertsätzen des Modells (130) des Herz-Kreislauf-Systems und den entsprechenden Messwertsätzen unterhalb einer Unsicherheit des entsprechenden Messwertsatzes liegt.

9. Verarbeitungssystem (110) nach einem der Ansprüche 1 bis 8, wobei das Verarbeitungssystem weiter dazu konfiguriert ist, nach der iterativen Modifikation:

die Vielzahl von Ausgabewertsätzen des Modells (130) des Herz-Kreislauf-Systems auf Basis des modifizierten Wertsatzes für jeden der Vielzahl von Modellparametern zu bestimmen; und
eine oder mehrere Herzfunktionskurven auf Basis der bestimmten Vielzahl von Ausgabewertsätzen zu erzeugen.

10. Computerimplementiertes Verfahren (1100) zum Schätzen eines linksventrikulären Fülldrucks eines Subjekts, wobei das computerimplementierte Verfahren umfasst:

Erhalten (1110) eines Modells (130) eines Herz-Kreislauf-Systems, das eine Vielzahl von Modellparametern verwendet, um Ausgabedaten zu erzeugen, die eine Vielzahl von Ausgabewertsätzen umfassen, die jeweils einer anderen nichtinvasiv messbaren Eigenschaft der Herzfunktion zugeordnet sind, wobei:

jeder Ausgabewertsatz mindestens einen Wert der zugeordneten nichtinvasiv messbaren Eigenschaft der Herzfunktion enthält; und
die Vielzahl von Modellparametern den Ziel-Herzparameter beinhaltet und/oder die Ausgabedaten einen weiteren Ausgabewertsatz für den Ziel-Herzparameter beinhalten;

Erhalten (1120) von Herzdaten (140), die eine Vielzahl von Messwertsätzen umfassen, wobei jeder Messwertsatz einem anderen Ausgabewertsatz zugeordnet ist und einen oder mehrere Messwerte für die nichtinvasiv messbare Eigenschaft, die dem zugeordneten Ausgabewertsatz zugeordnet ist, umfasst;
iteratives Modifizieren (1130) der Werte der Vielzahl von Modellparametern des Modells, um dadurch eine Differenz zwischen der Vielzahl von Ausgabewertsätzen der Ausgabedaten und den entsprechenden Messwertsätzen in den Herzdaten zu modifizieren, bis ein oder mehrere vorbestimmte Kriterien erfüllt sind, wobei die Werte der Vielzahl von Modellparametern des Modells iterativ modifiziert werden durch:

Aufteilen der Vielzahl von Modellparametern in eine Vielzahl von Sätzen eines oder mehrerer Modellparameter;
Festlegen eines Anfangswertsatzes für jeden der Vielzahl von Modellparametern;
Durchführen der folgenden iterativen Schritte für jeden Satz eines oder mehrerer Modellparameter nacheinander:

Bestimmen eines oder mehrerer Ausgabewertsätze des Modells des Herz-Kreislauf-Systems auf Basis eines aktuellen Wertsatzes für mindestens den Satz Modellparameter;
Berechnen eines Werts einer Kostenfunktion auf Basis des einen oder der mehreren bestimmten Ausgabewertsätze und der Messwertsätze, die dem einen oder den mehreren Ausgabewertsätzen zugeordnet sind; und
Anpassen des Wertsatzes für jeden Modellparameter in dem Satz Modellparameter auf Basis des berechneten Werts der Kostenfunktion,

wobei die iterativen Schritte wiederholt werden, bis eine vordefinierte Konvergenzbedingung erfüllt ist; und

nach der iterativen Modifikation, entweder (1140):

wenn die Vielzahl von Modellparametern den Ziel-Herzparameter beinhaltet, Definieren des Satzes eines oder mehrerer Werte des modifizierten Ziel-Herzparameters als den geschätzten Satz eines oder mehrerer Werte für den Ziel-Herzparameter; oder
wenn die Ausgabedaten den weiteren Ausgabewertsatz für den Ziel-Herzparameter beinhalten, Definieren des weiteren Ausgabewertsatzes als den geschätzten Satz eines oder mehrerer Werte für den Ziel-Herzparameter.

11. Computerprogrammprodukt, das Codemittel umfasst, die, wenn sie auf einem Computer ausgeführt werden, der ein Verarbeitungssystem aufweist, bewirken, dass das Verarbeitungssystem alle Schritte des Verfahrens (1100) nach Anspruch 10 durchführt.

**Revendications**

1. Système de traitement (110) permettant d'estimer un ensemble d'une ou plusieurs valeurs relatives à un paramètre cardiaque cible d'un sujet, le système de traitement étant configuré pour :

   obtenir un modèle (130) d'un système cardiovasculaire qui utilise une pluralité de paramètres de modèle pour produire des données de sortie comprenant une pluralité d'ensembles de valeurs de sortie, chacun associé à une propriété différente mesurable de manière non invasive d'une fonction cardiaque, dans lequel :

   chaque ensemble de valeurs de sortie contient au moins une valeur de la propriété associée mesurable de manière non invasive d'une fonction cardiaque ; et
   la pluralité de paramètres du modèle inclut le paramètre cardiaque cible et/ou les données de sortie incluent un ensemble de valeurs de sortie supplémentaire relatif au paramètre cardiaque cible ;

   obtenir des données cardiaques (140) comprenant une pluralité d'ensembles de valeurs mesurées, dans lequel chaque ensemble de valeurs mesurées est associé à un ensemble de valeurs de sortie différent et comprend une ou plusieurs valeurs mesurées relatives à la propriété mesurable de manière non invasive associée à l'ensemble de valeurs de sortie associé ;
   modifier de manière itérative les valeurs de la pluralité de paramètres du modèle afin de modifier ainsi une différence entre la pluralité d'ensembles de valeurs de sortie des données de sortie et les ensembles de valeurs mesurées correspondants dans les données cardiaques jusqu'à ce qu'un ou plusieurs critères prédéterminés soient satisfaits, dans lequel le système de traitement est configuré pour modifier de manière itérative les valeurs de la pluralité de paramètres du modèle en :

   divisant la pluralité de paramètres du modèle en une pluralité d'ensembles d'un ou plusieurs paramètres du modèle ;
   définissant un ensemble de valeurs initial relatif à chacun de la pluralité de paramètres du modèle ;
   effectuant, pour chaque ensemble d'un ou plusieurs paramètres du modèle à tour de rôle, les étapes itératives de :

   détermination d'un ou plusieurs ensembles de valeurs de sortie du modèle du système cardiovasculaire sur la base d'un ensemble de valeurs actuelles associé au moins à l'ensemble de paramètres du modèle ;
   calcul d'une valeur d'une fonction de coût sur la base des un ou plusieurs ensembles de valeurs de sortie déterminés et des ensembles de valeurs mesurées associés aux un ou plusieurs ensembles de valeurs de sortie ; et
   ajustement de l'ensemble de valeurs relatif à chaque paramètre du modèle dans l'ensemble de paramètres du modèle sur la base de la valeur calculée de la fonction de coût,

   dans lequel les étapes itératives sont répétées jusqu'à ce qu'une condition de convergence prédéfinie soit satisfaite ; et

   suite à la modification itérative :

   si la pluralité de paramètres du modèle inclut le paramètre cardiaque cible, définir l'ensemble d'une ou plusieurs valeurs du paramètre cardiaque cible modifié comme l'ensemble estimé d'une ou plusieurs valeurs relatives au paramètre cardiaque cible ; ou
   si les données de sortie incluent l'ensemble de valeurs de sortie supplémentaire relatif au paramètre cardiaque cible, définir l'ensemble de valeurs de sortie supplémentaire comme l'ensemble estimé d'une ou plusieurs valeurs relatives au paramètre cardiaque cible.

2. Système de traitement (110) selon la revendication 1, dans lequel le paramètre cardiaque cible est une pression ventriculaire gauche.

3. Système de traitement (110) selon la revendication 1 ou 2, dans lequel la pluralité de propriétés mesurables de manière non invasive comprend un volume ventriculaire gauche et au moins une propriété de pression périphérique.

4. Système de traitement (110) selon l'une quelconque des revendications 1 à 3, dans lequel la pluralité d'indicateurs mesurables de manière non invasive comprend au moins l'un parmi : un flux valvulaire aortique, un flux valvulaire mitral et/ou une chronologie d'un événement d'un cycle cardiaque.

5. Système de traitement (110) selon l'une quelconque des revendications 1 à 4, dans lequel le système de traitement est configuré pour :

diviser la pluralité de paramètres du modèle en une pluralité d'ensembles d'un ou plusieurs paramètres du modèle en divisant la pluralité de paramètres du modèle en un premier ensemble de paramètres du modèle et en un second ensemble de paramètres du modèle ;
modifier un ensemble de valeurs relatif à chacun du premier ensemble de paramètres du modèle en :

déterminant la pluralité d'ensembles de valeurs de sortie du modèle (130) du système cardiovasculaire sur la base d'un ensemble de valeurs actuelles relatif à chacun de la pluralité de paramètres du modèle ;
calculant une valeur de la fonction de coût sur la base de la pluralité déterminée d'ensembles de valeurs de sortie ;
ajustant l'ensemble de valeurs relatif à chacun du premier ensemble de paramètres du modèle sur la base de la valeur calculée de la fonction de coût ; et
répétant de manière itérative les étapes de détermination de la pluralité d'ensembles de valeurs de sortie, de calcul de la valeur de la fonction de coût et d'ajustement de l'ensemble de valeurs relatif à chacun du premier ensemble de paramètres du modèle jusqu'à ce qu'une première condition de convergence prédéfinie soit satisfaite ;

modifier un ensemble de valeurs relatif à chacun du second ensemble de paramètres du modèle en :

déterminant la pluralité d'ensembles de valeurs de sortie du modèle du système cardiovasculaire sur la base d'un ensemble de valeurs actuelles relatif à chacun de la pluralité de paramètres du modèle ;
calculant une valeur de la fonction de coût sur la base de la pluralité déterminée d'ensembles de valeurs de sortie ;
ajustant l'ensemble de valeurs relatif à chacun du second ensemble de paramètres du modèle sur la base de la valeur calculée de la fonction de coût ; et
répétant de manière itérative les étapes de détermination de la pluralité d'ensembles de valeurs de sortie, de calcul de la valeur de la fonction de coût et d'ajustement de l'ensemble de valeurs relatif à chacun du second ensemble de paramètres du modèle jusqu'à ce qu'une seconde condition de convergence prédéfinie soit satisfaite ; et
répéter de manière itérative les étapes de modification d'un ensemble de valeurs relatif à chacun du premier ensemble de paramètres du modèle et de modification d'un ensemble de valeurs relatif à chacun du second ensemble de paramètres du modèle jusqu'à ce que les un ou plusieurs critères prédéterminés soient satisfaits.

6. Système de traitement (110) selon la revendication 5, dans lequel la pluralité de paramètres du modèle inclut le paramètre cardiaque cible et le système de traitement est configuré pour diviser la pluralité de paramètres du modèle en un premier ensemble de paramètres du modèle et en un second ensemble de paramètres du modèle en :

définissant le premier ensemble de paramètres du modèle de façon à ce qu'il ne comprenne que le paramètre cardiaque cible ; et
définissant le second ensemble de paramètres du modèle de façon à ce qu'il comprenne les autres paramètres du modèle.

7. Système de traitement (110) selon l'une quelconque des revendications 1 à 6, dans lequel le système de traitement est configuré pour diviser la pluralité de paramètres du modèle en une pluralité d'ensembles d'un ou plusieurs paramètres du modèle en :

effectuant une analyse de sensibilité sur le modèle du système cardiovasculaire ; et
divisant la pluralité de paramètres du modèle en la pluralité d'ensembles d'un ou plusieurs paramètres du modèle sur la base de l'analyse de sensibilité.

8. Système de traitement (110) selon l'une quelconque des revendications 1 à 7, dans lequel les un ou plusieurs critères prédéterminés comprennent au moins l'un parmi : une détermination selon laquelle une différence dans un ensemble de valeurs de chacun de la pluralité de paramètres du modèle entre une itération en cours et une itération immédiatement précédente est inférieure à un seuil prédéterminé ; une détermination selon laquelle une différence dans la valeur de la fonction de coût entre une itération en cours et une itération immédiatement précédente est inférieure à un seuil prédéterminé ; une détermination selon laquelle un nombre d'itérations a dépassé un seuil prédéterminé ; une détermination selon laquelle une durée passée à effectuer la modification itérative est supérieure à une durée prédéterminée et/ou une détermination selon laquelle une différence entre chacun de la pluralité d'ensembles de valeurs de sortie du modèle (130) du système cardiovasculaire et les ensembles de valeurs mesurées correspondants est inférieure à une incertitude de l'ensemble de valeurs mesurées correspondant.

9. Système de traitement (110) selon l'une quelconque des revendications 1 à 8, dans lequel le système de traitement est en outre configuré pour, suite à la modification itérative :

   déterminer la pluralité d'ensembles de valeurs de sortie du modèle (130) du système cardiovasculaire sur la base de l'ensemble de valeurs modifiées relatif à chacun de la pluralité de paramètres du modèle ; et
   générer une ou plusieurs courbes d'une fonction cardiaque sur la base de la pluralité déterminée d'ensembles de valeurs de sortie.

10. Procédé mis en œuvre par ordinateur (1100) permettant d'estimer une pression de remplissage ventriculaire gauche d'un sujet, le procédé mis en œuvre par ordinateur comprenant :

   l'obtention (1110) d'un modèle (130) d'un système cardiovasculaire qui utilise une pluralité de paramètres du modèle pour produire des données de sortie comprenant une pluralité d'ensembles de valeurs de sortie, chacun associé à une propriété différente mesurable de manière non invasive d'une fonction cardiaque, dans lequel :

      chaque ensemble de valeurs de sortie contient au moins une valeur de la propriété associée mesurable de manière non invasive d'une fonction cardiaque ; et
      la pluralité de paramètres du modèle inclut le paramètre cardiaque cible et/ou les données de sortie incluent un ensemble de valeurs de sortie supplémentaire relatif au paramètre cardiaque cible ;

   l'obtention (1120) de données cardiaques (140) comprenant une pluralité d'ensembles de valeurs mesurées, dans lequel chaque ensemble de valeurs mesurées est associé à un ensemble de valeurs de sortie différent et comprend une ou plusieurs valeurs mesurées relatives à la propriété mesurable de manière non invasive associée à l'ensemble de valeurs de sortie associé ;
   la modification de manière itérative (1130) des valeurs de la pluralité de paramètres du modèle afin de modifier ainsi une différence entre la pluralité d'ensembles de valeurs de sortie des données de sortie et les ensembles de valeurs mesurées correspondants dans les données cardiaques jusqu'à ce qu'un ou plusieurs critères prédéterminés soient satisfaits, dans lequel les valeurs de la pluralité de paramètres du modèle sont modifiées de manière itérative en :

      divisant la pluralité de paramètres du modèle en une pluralité d'ensembles d'un ou plusieurs paramètres du modèle ;
      définissant un ensemble de valeurs initial relatif à chacun de la pluralité de paramètres du modèle ;
      effectuant, pour chaque ensemble d'un ou plusieurs paramètres du modèle à tour de rôle, les étapes itératives de :

         détermination d'un ou plusieurs ensembles de valeurs de sortie du modèle du système cardiovasculaire sur la base d'un ensemble de valeurs actuelles associé au moins à l'ensemble de paramètres du modèle ;
         calcul d'une valeur d'une fonction de coût sur la base des un ou plusieurs ensembles de valeurs de sortie déterminés et des ensembles de valeurs mesurées associés aux un ou plusieurs ensembles de valeurs de sortie ; et
         ajustement de l'ensemble de valeurs relatif à chaque paramètre du modèle dans l'ensemble de paramètres du modèle sur la base de la valeur calculée de la fonction de coût,

      dans lequel les étapes itératives sont répétées jusqu'à ce qu'une condition de convergence prédéfinie soit satisfaite ; et

   suite à la modification itérative, (1140) :

si la pluralité de paramètres du modèle inclut le paramètre cardiaque cible, définition de l'ensemble d'une ou plusieurs valeurs du paramètre cardiaque cible modifié comme l'ensemble estimé d'une ou plusieurs valeurs relatives au paramètre cardiaque cible ; ou

si les données de sortie incluent l'ensemble de valeurs de sortie supplémentaire relatif au paramètre cardiaque cible, définition de l'ensemble de valeurs de sortie supplémentaire comme l'ensemble estimé d'une ou plusieurs valeurs relatives au paramètre cardiaque cible.

11. Produit de programme informatique comprenant des moyens de code qui, lorsqu'ils sont exécutés sur un ordinateur pourvu d'un système de traitement, amènent le système de traitement à réaliser toutes les étapes du procédé (1100) selon la revendication 10.

FIG. 1

FIG. 2

$\Delta_{rel}V_{LV}$ $\Delta_{rel}P_{AO}$

$\Delta_{rel}Q_{AO}$ $\Delta_{rel}Q_{MV}$

FIG. 3

$\Delta p_{sys}$

$\Delta p_{dia}$

FIG. 4

500

Set Initial Value(s) for model parameters ⌐510

Determine output value set(s) ⌐520

Perform Cost Function ⌐530

Criteria Met? ⌐540

No

Yes

Modify value(s) of model parameters ⌐550

Estimate value(s) for target cardiac parameter ⌐560

FIG. 5

600

610

Divide model parameters
into two sets

620

Set Initial Value(s) for
model parameters

630

Modify first set of model
parameters

640

Modify second set of
model parameters

650

No — Criteria
Met?

Yes

660

Estimate value(s) for target cardiac
parameter

FIG. 6

700

1.50 –

1.25 –

1.00 –

0.75 –

0.50 –

0.25 –

0.00 –

r_p   r_d   c   r_mv   p_la   e_min   e_max   m1   m2   tau1   tau2   vlv_0

S1
ST

## FIG. 7

630

Determine output value set(s) — 631

Perform cost function — 632

Condition Met? — 633

No

Modify value(s) of first set of model parameters — 634

Yes

640

## FIG. 8

FIG. 9

1000

1010
Divide model parameters into N sets

1020
Set Initial Value(s) for model parameters

1030
Modify Zth set of model parameters

1040
Criteria Met?

No

Yes

1055 Z +1

1050
Z=N?

No

Yes

1060
Estimate value(s) for target cardiac parameter

FIG. 10

1100

1110
Obtain CVS model

1120
Obtain cardiac data

Iteratively modify parameters of CVS model — 1130

Define value(s) for target cardiac parameter — 1140

FIG. 11

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 3043276 A2

**Non-patent literature cited in the description**

- **WANG et al.** Monitoring of the central blood pressure waveform via a conformal ultrasonic device. *Nat Biomed Eng*, 2018, vol. 2, 687-695